(19) Europäisches Patentamt / European Patent Office / Office européen des brevets

(11) **EP 2 899 224 A1**

(12) **EUROPEAN PATENT APPLICATION**
published in accordance with Art. 153(4) EPC

(43) Date of publication:
**29.07.2015 Bulletin 2015/31**

(21) Application number: **13838248.6**

(22) Date of filing: **18.09.2013**

(51) Int Cl.:
**C08G 81/02** (2006.01)    **C08F 251/00** (2006.01)
**C08K 5/00** (2006.01)    **C08L 51/02** (2006.01)

(86) International application number:
**PCT/JP2013/075106**

(87) International publication number:
**WO 2014/046106 (27.03.2014 Gazette 2014/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.09.2012 JP 2012208416**

(71) Applicant: **Sanyo Chemical Industries, Ltd. Kyoto-shi, Kyoto 605-0995 (JP)**

(72) Inventors:
• **UEDA, Yusuke**
  **Kyoto-shi**
  **Kyoto 605-0995 (JP)**
• **KANDA, Yoichi**
  **Kyoto-shi**
  **Kyoto 605-0995 (JP)**

(74) Representative: **Vossius & Partner**
**Patentanwälte Rechtsanwälte mbB**
**Siebertstrasse 3**
**81675 München (DE)**

(54) **AQUEOUS LIQUID ABSORBING RESIN, AQUEOUS LIQUID ABSORBING COMPOSITION, AND ABSORBENT BODY AND ABSORBENT ARTICLE USING SAME**

(57)    An absorbent resin and an absorbent composition that use a large amount of material derived from plants, have excellent aqueous liquid absorbing capability, and have excellent absorbing capability in a compressed state due to having a favorable gel elastic modulus. An absorbent resin made by bonding (A1) an oxidized polysaccharide having a carboxyl group that may be neutralized by a neutralizing agent, and/or (A2) a crosslinked compound thereof with (B1) a poly(meth) acrylic acid where at least one of the carboxyl groups may be neutralized by a neutralizing agent, and/or (B2) a crosslinked compound thereof; or an absorbent composition made by blending (A1) and/or (A2) and (B1) and/or the (B2) ; wherein the weight average molecular weight of (A1) is 2, 000 to 10, 000, 000, the acid value of (A1) is 65 to 524 mgKOH/g, and if (A1) has a carboxyl group that has been neutralized by a neutralizing agent, the acid value prior to neutralizing is 65 to 524 mgKOH/g.

**Description**

TECHNICAL FIELD

The present invention relates to an aqueous liquid absorbing resin and an aqueous liquid absorbing composition as well as an aqueous liquid absorbing material and an aqueous liquid absorbing article each using them.

BACKGROUND ART

Conventionally, a hydrophilic crosslinked polymer called a water-absorbing resin is known as a particulate or granular absorbent having an absorption capacity for an Aqueous liquid. Known examples of such a water absorbing resin include various resins such as a crosslinked polyacrylic acid salt, a crosslinked copolymer of acrylic acid, a salt thereof, and another monomer, an isobutylene-maleic anhydride crosslinked copolymer, a polyvinyl alcohol-(meth)acrylic acid copolymer, modified polyethylene oxide, and modified polyvinyl alcohol, and these have been mainly used for hygienic materials such as disposable diapers and sanitary napkins.

In recent years, attempts have been made to use a plant-derived feedstock for a water absorbing resin, and starch-acrylate copolymers are known. As described in Non-Patent Document 1, there is a problem that water absorptivity decreases when starch is mixed in a large amount though water absorptivity increases when the content of starch is small. This is believed to be because starch has no ionic groups and the starch itself has no capacity to absorb water. Patent Document 1 discloses, as a water absorbing powder, a modified starch composition obtained by slightly oxidizing starch to make it have 0.05 to 0.5 wt% of carboxyl groups and then crosslinking it. The powder obtained by this method is problematic in that its water absorption capacity is low due to its small content of carboxyl groups and it demonstrates low water absorption capacity under pressure due to the low modulus of starch.

PRIOR ART DOCUMENTS

PATENT DOCUMENT

Patent Document 1: Specification of European Patent Application Publication No. 0489424

NON-PATENT DOCUMENT

Non-Patent Document 1: F. MASUDA, Chemical Economy & Engineering Review; November 1983, 19p (No. 173)

SUMMARY OF THE INVENTION

PROBLEMS TO BE SOLVED BY THE INVENTION

The present invention was devised in view of the above-described problems and an object of the present invention is to provide an absorbent resin and an absorbent composition in which much plant-derived feedstock is used and which are excellent in absorption capacity for an aqueous liquid and also excellent in absorption capacity for an aqueous liquid under pressure due to its possession of good gel elastic modulus.

SOLUTIONS TO THE PROBLEMS

As a result of earnest studies for achieving the above object, the present inventors have accomplished the present invention. Specifically, the present invention includes:

(C) an aqueous liquid absorbing resin (hereinafter alternatively merely referred to as absorbent resin) comprising (A1) an oxidized polysaccharide having a carboxyl group optionally neutralized by a neutralizing agent and/or (A2) a crosslinked product thereof and (B1) a poly(meth)acrylic acid with at least one of the carboxyl groups thereof optionally being neutralized by a neutralizing agent and/or (B2) a crosslinked product thereof, the (A1) and/or the (A2) being bonded with the (B1) and/or the (B2), wherein the weight average molecular weight of the (A1) is 2,000 to 10,000,000, the acid value of the (A1) is 65 to 524 mgKOH/g when the (A1) has no carboxyl groups neutralized by a neutralizing agent, and the acid value of the (A1) prior to its neutralization is 65 to 524 mgKOH/g when the (A1) has a carboxyl group neutralized by a neutralizing agent;

(P-1) an absorbent resin particle comprising the absorbent resin (C) and a hydrophobic substance (E), wherein the (E) is a compound having at least one monovalent aliphatic hydrocarbon group having 8 to 26 carbon atoms and having at least one functional group capable of forming a hydrogen bond with a carboxyl group, and the (E) is present in the inside of the absorbent resin particle in an amount of 0.01 to 10.0 wt% based on the weight of the absorbent resin and on the surface of the absorbent resin particle in an amount of 0.001 to 1.0 wt%;

(D) an aqueous liquid absorbing composition (hereinafter alternatively merely referred to as absorbent composition) comprising (A1) an oxidized polysaccharide having a carboxyl group optionally neutralized by a neutralizing agent and/or (A2) a crosslinked product thereof and (B1) a poly (meth) acrylic acid with at least one of the carboxyl groups thereof optionally being neutralized by a neutralizing agent and/or (B2) a crosslinked product thereof, wherein the weight average molecular weight of the (A1) is 2, 000 to 10, 000, 000, the acid value of the (A1) is 65 to 524 mgKOH/g when the (A1) has no carboxyl groups neutralized by a neutralizing agent, and the acid value of the (A1) prior to its neutralization is 65 to 524 mgKOH/g when the (A1) has a carboxyl group neutralized by a neutralizing agent;

(P-2) an absorbent composition particle comprising the absorbent composition (D) and a hydrophobic substance (E), wherein the (E) is a compound having at least one monovalent aliphatic hydrocarbon group having 8 to 26 carbon atoms and having at least one functional group capable of forming a hydrogen bond with a carboxyl group, and the (E) is present in the inside of the absorbent composition particle in an amount of 0.01 to 10.0 wt% based on the weight of the absorbent composition particle and on the surface of the absorbent composition particle in an amount of 0.001 to 1.0 wt%;

an absorbent material produced using the absorbent resin (C), the absorbent composition (D), the absorbent resin particle (P-1) and/or the absorbent composition particle (P-2), and at least one species selected from the group consisting of a fiber, a nonwoven fabric, and a woven fabric; and an absorbent article produced using the absorbent material.

## ADVANTAGES OF THE INVENTION

The absorbent resin and the absorbent composition of the present invention use much plant-derived feedstock, and the resin and the composition are excellent in absorption capacity for an aqueous liquid and also excellent in absorption capacity under pressure due to their possession of good gel elastic modulus.

## BRIEF DESCRIPTION OF THE DRAWINGS

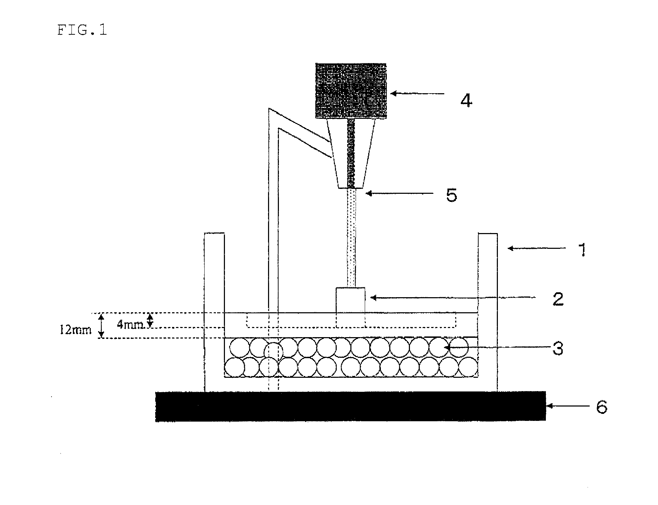

Fig. 1 is a front sectional view schematically illustrating the entirety of a device for measuring an absorption amount by a method of measuring a swollen volume.

## MODE FOR CARRYING OUT THE INVENTION

The absorbent resin (C) of the present invention is an absorbent resin comprising (A1) an oxidized polysaccharide having a carboxyl group optionally neutralized by a neutralizing agent (hereinafter alternatively merely referred to as oxidized polysaccharide (A1) or the (A1)) and/or (A2) a crosslinked product thereof (hereinafter alternatively merely referred to as crosslinked product (A2) or the (A2)) and (B1) a poly(meth)acrylic acid with at least one of the carboxyl groups thereof optionally being neutralized by a neutralizing agent (hereinafter alternatively merely referred to as poly(meth)acrylic acid (B1) or the (B1)) and/or (B2) a crosslinked product thereof (hereinafter alternatively merely referred to as crosslinked product (B2) or the (B2)), both (namely, the (A1) and/or the (A2)) being bonded with the (B1) and/or the (B2), wherein the weight average molecular weight of the (A1) is 2, 000 to 10, 000, 000, the acid value of the (A1) is 65 to 524 mgKOH/g when the (A1) has no carboxyl groups neutralized by a neutralizing agent, and the acid value of the (A1) prior to its neutralization is 65 to 524 mgKOH/g when the (A1) has a carboxyl group neutralized by a neutralizing agent.

Examples of the oxidized polysaccharide (A1) in the present invention include oxidized product of starch, cellulose, glycogen, chitin, chitosan, agarose, carrageenan, heparin, hyaluronic acid, pectin, xyloglucan, etc. and such oxidized products having a carboxyl group optionally neutralized by a neutralizing agent. When a plurality of carboxyl groups are possessed, at least some of them may have been neutralized. Among these, an oxidized starch, an oxidized cellulose, and these oxidized products having a carboxyl group optionally neutralized by a neutralizing agent are preferred in view of reactivity. As for the oxidized polysaccharide (A1), a single species thereof may be used alone or two or more species thereof may be used in combination.

Examples of the starch used for the oxidized starch include corn starch, potato starch, tapioca starch, wheat starch, sweet potato starch, and rice starch. In addition, modified starches using these starches as a raw material, such as water-soluble starch, oxidized starch, carboxylated starch, phosphorylated starch, cationized starch, aldehyded starch, acetylated starch, alkyl etherified starch, hydroxy etherified starch, allyl etherified starch, and vinyl etherified starch, may also be used. Examples of the water-soluble starch include a hydrolyzed starch obtained by acid hydrolysis. Examples of the oxidized starch include commercially available oxidized starches treated with hypochlorite or the like. Commercially available oxidized starches are starches in which part of the hydroxyl groups in a glucopyranose, which is a constituent unit of starch, are carboxylated or aldehyded.

Examples of the cellulose used for the oxidized cellulose include cotton, wood-derived pulp, bacteria cellulose, lignocellulose, regenerated cellulose (for example, Cellophane and regenerated fiber), and microcrystalline cellulose.

As a method for obtaining the oxidized polysaccharide (A1) a commonly known method may be used. For example, there may be used a method of performing oxidation by using a catalyst based on a noble metal such as palladium and an oxygen-containing gas described in JP-A-62-247837 and a method of performing oxidation by using a manganese oxide or a salt thereof described in JP-A-10-195101.

The acid value of the oxidized polysaccharide (A1) is from 65 to 524 mgKOH/g, and in view of absorption capacity, preferably from 66 to 524 mgKOH/g, more preferably from 70 to 524 mgKOH/g, and even more preferably from 100 to 524 mgKOH/g. If the acid value is less than 65 mgKOH/g, the ionic group density of the absorbent resin formed becomes lower, giving rise to bad absorption capacity. If the lower limit is 66 mgKOH/g, the absorption capacity is good, if the lower limit is 70 mgKOH/g, the absorption capacity is better, and if the lower limit is 100 mgKOH/g, the absorption capacity is further improved.

<Measurement Method of Acid Value>

Into a 300 ml beaker, 1.00 g of a non-neutralized oxidized polysaccharide is precisely weighed and after adding pure water to make 100 g, dissolved with stirring at 95°C for 15 minutes to prepare a measurement solution. The measurement solution is first titrated to pH 10 with an aqueous 1/50 N KOH solution and then titrated to pH 2.7 with an aqueous 1/20 N HCl solution. On the other hand, as a blank, 100 g of pure water is titrated by the same method. The amount of the aqueous 1/20 N HCl solution necessary for titration from pH 10 to pH 2.7 in the blank is subtracted from the amount of the aqueous 1/20 N HCl solution necessary for titration from pH 10 to pH 2.7 in the measurement solution having 1.00 g of an oxidized polysaccharide dissolved therein, and from the obtained value, the acid value is calculated.

Incidentally, in the case where part or all of the carboxyl groups in the oxidized polysaccharide (A1) to be measured for the acid value have been neutralized by a neutralizing agent to make a salt form, an aqueous 1 wt% solution of the oxidized polysaccharide (A1) is prepared, mixed with a cation exchange resin (DOWEX50W-X8) and stirred for 15 minutes, thereby effecting cation exchange and conversion to a non-neutralization type oxidized polysaccharide, and thereafter, the cation exchange resin is removed by filtration, the aqueous solution is dried up, and then the acid value is measured in the same manner as above.

The weight average molecular weight (hereinafter, simply referred to as Mw) of the oxidized polysaccharide (A1) is from 2,000 to 10,000,000, and in view of resin strength and absorption capacity, preferably from 5,000 to 5,000,000, more preferably from 10,000 to 1,000,000. If the Mw is less than 2, 000, elution to the absorbing liquid is increased and, in turn, the absorption capacity is impaired, whereas if it exceeds 10,000,000, the absorption capacity is impaired.

The Mw of the (A1) can be measured by gel permeation chromatography (GPC) under the following conditions:
Solvent: an aqueous 30 vol% methanol solution containing 0.5 wt% of sodium acetate

Sample concentration: 2.5 mg/ml
Column used: Guardcolumn PWXL + TSKgel G6000 PWXL + TSKgel G3000 PWXL, manufactured by Tosoh Corporation
Column temperature: 40°C

Examples of the neutralizing agent for neutralizing a carboxyl group contained in the oxidized polysaccharide (A1) include ammonia, an amine compound having 1 to 20 carbon atoms, and an alkali metal hydroxide (e.g., sodium hydroxide, potassium hydroxide and lithium hydroxide).

Examples of the amine compound having 1 to 20 carbon atoms include primary amines such as monomethylamine, monoethylamine, monobutylamine and monoethanolamine, secondary amines such as dimethylamine, diethylamine, dibutylamine, diethanolamine, diisopropanolamine and methylpropanolamine, and tertiary amines such as trimethylamine, triethylamine, dimethylethylamine, dimethylmonoethanolamine and triethanolamine.

Among these neutralizing agents, an alkali metal hydroxide is preferred in view of suppression of coloration after neutralization. As for the neutralizing agent, a single species thereof may be used alone or two or more species thereof may be used in combination.

The methods for obtaining (A2) a crosslinked product of the oxidized polysaccharide (A1) include a method of crosslinking the (A1), a method using a crosslinked polysaccharide as a feedstock polysaccharide to be used for oxidation, and a method using these methods in combination.

The method of crosslinking the (A1) includes a method of performing the crosslinking by using the following (k1) to (k6) :

(1) (k1) a polyhydric (preferably from dihydric to tetrahydric) alcohol having 2 to 6 carbon atoms (e.g., ethylene glycol, diethylene glycol, triethylene glycol, 1,3-propanediol, dipropylene glycol, glycerin, diglycerin, 1,4-butanediol, 1,5-pentanediol, 1,6-hexanediol and sorbitol);

(2) (k2) a polyvalent (preferably from divalent to tetravalent) glycidyl ether having 8 to 21 carbon atoms [e.g., ethylene glycol diglycidyl ether, polyethylene glycol (polymerization degree: from 2 to 4) diglycidyl ether, glycerin polyglycidyl ether, diglycerin polyglycidyl ether, propylene glycol diglycidyl ether, polypropylene glycol (polymerization degree: from 2 to 3) diglycidyl ether, sorbitol polyglycidyl ether, neopentyl glycol diglycidyl ether and 1,6-hexanediol diglycidyl ether];

(3) (k3) a polyvalent (preferably from divalent to tetravalent) amine having 2 to 6 carbon atoms (e.g., ethylenediamine, diethylenetriamine, triethylenetetramine and polyethyleneimine) ;

(4) (k4) an alkanolamine having 2 to 8 carbon atoms (e.g., monoethanolamine, diethanolamine, monopropanolamine and dibutanolamine) ;

(5) (k5) a polyvalent (preferably from divalent to tetravalent) aziridine compound having 6 to 12 carbon atoms [e.g., 2,2-bishydroxymethylbutanol-tris[3-(1-aziridinyl)propionate ], 4,4'-bis(ethyleneiminccarbonylamino)diphenylmethane and 1,6-bis(ethyleneiminocarbonylamino)hexane]; and

(6) (k6) an alkylene carbonate having 3 to 4 carbon atoms (e.g., 1,3-dioxolan-2-one, 4-methyl-1,3-dioxolan-2-one and 1,3-dioxan-2-one).

Among these, in view of crosslinking density, (k1), (k2), (k3) and (k4) are preferred, (k2) and (k3) are more preferred, and (k2) is particularly preferred. As for (k1) to (k6), a single species thereof may be used alone or two or more species thereof may be used in combination.

The polysaccharide can be crosslinked by any known method and may be crosslinked using a crosslinking agent or may be crosslinked by radiation (radiation such as y-ray, x-ray or electron beam) and/or heat.

Examples of the crosslinking agent used for the crosslinking of the polysaccharide include a methylol group-containing urea-based or melamine-based compound (e.g., dimethylolurea, trimethylolmelamine, dimethylolethyleneurea and dimethyloldihydroxyethyleneurea), a polycarboxylic acid (e.g., citric acid, tricarballylic acid and 1,2,3,4-butanetetracarboxylic acid), and the above-described (k1) to (k5). These may be used singly or two or more thereof may be used in combination.

The conditions for the crosslinking reaction of the (A1) vary depending on the crosslinking agent used, but the conditions (for example, from 50 to 100°C, and from 10 minutes to 2 hours) usually employed for the reaction between a functional group contained in a polysaccharide or the (A1) and a functional group contained in the crosslinking agent may be applied.

The production method of (B1) a poly(meth)acrylic acid for use in the present invention is not particularly limited and includes, for example, a method of subjecting a (meth)acrylic acid monomer optionally neutralized by a neutralizing agent, to aqueous solution polymerization, suspension polymerization or reverse phase suspension polymerization by using a radical polymerization catalyst. A method of irradiating the monomer with radiation, an electron beam or an ultraviolet ray to initiate the polymerization may be also employed. Among these, aqueous solution polymerization is preferred, because an organic solvent or the like need not be used and this is advantageous in view of the production cost. From the standpoint that an absorbent resin having a large water-retention amount and a small amount of water-soluble components is obtained and the temperature control during polymerization is unnecessary, an aqueous solution adiabatic polymerization method is more preferred.

In the present invention, the polymerization conditions are not particularly limited, and, for example, the polymerization initiation temperature may be varied according to the kind of the catalyst used but is usually from 0 to 100°C, preferably from 5 to 80°C.

As for the polymerization catalyst used when performing polymerization by using a radical polymerization catalyst, a conventionally known catalyst may be used, and examples thereof include an azo compound (e.g., azobisisobutyronitrile, azobiscyanovaleric acid and 2,2'-azobis(2-amidinopropane)hydrochloride), an inorganic peroxide (e.g., hydrogen peroxide, ammonium persulfate, potassium persulfate and sodium persulfate), an organic peroxide (e.g., benzoyl peroxide, di-tert-butyl peroxide, cumene hydroperoxide, succinic peroxide and di(2-ethoxyethyl)peroxydicarbonate), a cerium compound (e.g., ammonium cerium(IV) nitrate and ammonium hexanitratocerium(IV)), and a redox catalyst (comprising a combination of a reducing agent such as alkali metal sulfite or bisulfite, ammonium sulfite, ammonium bisulfite and ascorbic acid, and an oxidizing agent such as alkali metal persulfate, ammonium persulfate, hydrogen peroxide and organic peroxide). These catalysts may be used singly or two or more thereof may be used in combination.

The amount of the radical polymerization catalyst used is usually from 0.0005 to 5 wt%, preferably from 0.001 to 2 wt%, based on the weight of the radical polymerizable monomer.

The method for neutralization using a neutralizing agent includes a method of using a (meth) acrylic acid monomer after neutralizing the monomer, a method of polymerizing a (meth) acrylic acid monomer and then neutralizing the polymer, and a method using these methods in combination.

1 Examples of the neutralizing agent for neutralizing a carboxyl group contained in the (B1) are the same as those of the neutralizing agent for neutralizing a carboxyl group contained in the oxidized polysaccharide (A1), and preferred examples are also the same.

The method for obtaining the crosslinked product (B2) includes, for example, a method of performing crosslinking by using the (k1) to the (k5), and a method of performing crosslinking by using (k7) a compound having a radical polymerizable double bond. As for each of the (k1) to the (k5) and the (k7), a single species thereof may be used alone or two or more species thereof may be used in combination.

The (k7) compounds having a radical polymerizable double bond include (k71) a compound having two or more radical polymerizable double bonds, and (k72) a compound having one functional group capable of reacting with a carboxyl group or a neutralized salt thereof and having at least one radical polymerizable double bond.

Examples of the (k71) compound having two or more radical polymerizable double bonds include bis(meth)acrylamides such as N,N'-methylenebisacrylamide; poly(meth)acrylates or poly(meth)allyl ethers of a polyhydric alcohol such as (poly)alkylene glycol, trimethylolpropane, glycerin, pentaerythritol and sorbitol; divinyl compounds such as divinylbenzene; and allyloxyalkanes such as tetraallyloxyethane and pentaerythritol triallyl ether.

Examples of the (k72) compound having one functional group capable of reacting with a carboxyl group or a neutralized salt thereof and having at least one radical polymerizable double bond include a hydroxyl group-containing radical polymerizable monomer such as hydroxyethyl (meth) acrylate and N-methylol(meth)acrylamide, and an epoxy group-containing radical polymerizable monomer such as glycidyl (meth) acrylate.

The conditions for the crosslinking reaction of the (B1) when using (k1) to (k5) as a crosslinking agent may vary depending on the crosslinking agent used, but the conditions (for example, at 50 to 100°C, for 10 minutes to 2 hours) usually employed for the reaction between a carboxyl group contained in the (B1) and a functional group contained in the crosslinking agent may be applied. In the case of using (k7) as a crosslinking agent, for example, a (meth) acrylic acid monomer and (k7) are mixed and polymerized under normal conditions (for example, at 0 to 100°C, and for 1 to 10 hours) and when (k72) is used, reaction is further performed under the conditions (for example, at 50 to 100°C, and for 10 minutes to 2 hours) usually employed for the reaction between a carboxyl group contained in (B1) and a functional group contained in (k72), whereby (B2) a crosslinked product of (B1) is obtained.

The absorbent resin (C) of the present invention is formed by binding (A1) an oxidized polysaccharide and/or (A2) a crosslinked product thereof, to (B1) a poly (meth) acrylic acid and/or (B2) a crosslinked product thereof.

Examples of the method for binding the (A1) and/or the (A2) to the (B1) and/or the (B2) include a method of binding these by using a binder, and a method of graft-polymerizing (B1) and/or (B2) to (A1) and/or (A2).

Examples of the binder include the (k1) polyhydric alcohol having 2 to 6 carbon atoms, the (k2) polyvalent glycidyl ether having 8 to 21 carbon atoms, the (k3) polyvalent amine having 2 to 6 carbon atoms, the (k4) alkanolamine having 2 to 8 carbon atoms, and the (k5) polyvalent aziridine compound having 6 to 12 carbon atoms. As for (k1) to (k5), a single species thereof may be used alone or two or more species thereof may be used in combination.

Among these, in view of binding density, (k1), (k2) and (k3) are preferred, (k2) and (k3) are more preferred, and (k2) is particularly preferred.

In view of the number of bonds and the water-retention amount, the amount of the binder used is preferably from 0.01 to 10 wt%, more preferably from 0. 02 to 5 wt%, based on the weight of the absorbent resin (C).

After mixing the binder, the binding reaction is completed by heating. The heating may be performed ether in a mixing apparatus or in a heating dryer. In view of prevention of thermal decomposition and the water-retention amount, the heating temperature is preferably from 30 to 250°C, more preferably from 50 to 180°C. The heating time may be varied according to the heating temperature, but in view of the reaction rate of binding reaction and the water-retention amount, the heating time is preferably from 5 to 180 minutes, more preferably from 15 to 90 minutes.

After mixing the binder, a known alkali agent may be added so as to accelerate the binding reaction. Incidentally, this mixing with alkali is differentiated from the neutralization with alkali in the usual production process of a water absorbent resin but can be performed in the same manner as in the neutralization step. Accordingly, in the case of performing neutralization, the neutralization and the alkali addition may be performed at the same time.

As for the alkali agent, known alkali agents (for example, Japanese Patent No. 3, 205, 168) may be used. Among these, in view of water absorption ability, lithium hydroxide, sodium hydroxide and potassium hydroxide are preferred, and sodium hydroxide is more preferred. As for the alkali agent, a single species thereof may be used alone or two or more species thereof may be used in combination.

The method for graft-polymerizing the (B1) and/or the (B2) to the (A1) and/or the (A2) is not particularly limited, and examples thereof include a method of performing the reaction of (B1) and/or (B2) in the presence of (A1) and/or (A2) by using an inorganic peroxide, an organic peroxide, a cerium compound or a redox catalyst as the radical polymerization catalyst, as described above.

Neutralization of a carboxyl group contained in the (A1), the (A2), the (B1) and the (B2) of the absorbent resin (C) by using a neutralizing agent may be performed before binding or after binding and obtaining the (C).

In view of irritation to skin or the like, the neutralization ratio is preferably less than 85%, more preferably from 65 to 75%, based on the total amount of carboxyl groups contained in the (B1) and the (B2) of the absorbent resin (C).

In view of irritation to skin or the like, the neutralization ratio is preferably from 65 to 75% based on the total amount of carboxyl groups contained in the (A1), the (A2), the (B1) and the (B2) of the absorbent resin (C).

From the standpoint of satisfying both the absorption capacity and the gel elastic modulus, the total amount of the (A1) and the (A2) bound in the absorbent resin (C) is preferably from 30 to 90 wt%, more preferably from 35 to 50%, based on the weight of the absorbent resin.

The absorbent composition (D) of the present invention contains, as essential components, the oxidized polysaccharide (A1) and/or the crosslinked product thereof (A2), and the poly(meth)acrylic acid (B1) and/or the crosslinked product thereof (B2).

The (D) may be obtained by mixing the (A1) and/or the (A2) with the (B1) and/or the (B2). The mixing method is not particularly limited and includes, for example, a method of mixing an Aqueous solution of the (A1) and/or a hydrous gel of the (A2) with an aqueous solution of the (B1) and/or a hydrous gel of the (B2) by means of a kneader, a universal mixer, a single-screw or twin-screw melt extruder, a mincing machine, a meat chopper or the like. Among these, a universal mixer, a single-screw or a twin-screw melt extruder, and a mincing machine are preferred, and a single-screw or twin-screw melt extruder and a mincing machine are more preferred.

The (D) may be also obtained by dry-blending the later-described particle composed of the (A1) and/or the (A2) and the resin particle composed of the (B1) and/or the (B2).

The absorbent composition (D) may further contain the absorbent resin (C). By virtue of containing the (C), it is more facilitated to satisfy both absorption capacity and higher gel elastic modulus.

Neutralization of a carboxyl group contained in the (A1), the (A2), the (B1) and the (B2) of the absorbent composition (D) by using a neutralizing agent may be performed before mixing or after mixing and obtaining (D).

In view of irritation to skin or the like, the neutralization ratio is preferably less than 85%, more preferably from 65 to 75%, based on the total amount of carboxyl groups contained in the (B1) and the (B2) of the absorbent composition (D).

In view of irritation to skin or the like, the neutralization ratio is preferably from 65 to 75% based on the total amount of carboxyl groups contained in the (A1), the (A2), the (B1) and the (B2) of the absorbent composition (D).

From the standpoint of satisfying both the absorption capacity and the gel elastic modulus, the total amount of the (A1) and the (A2) contained in the absorbent composition (D) and the (A1) and the (A2) bound in the absorbent resin (C) contained in the (D) is preferably from 30 to 90 wt%, more preferably from 35 to 50 wt% , based on the weight of the absorbent composition.

In the absorbent resin (C) and the absorbent composition (D) of the present invention, conventionally known additives (e.g., antiseptic, fungicide, antimicrobial, antioxidant, ultraviolet absorbing agent, colorant, fragrance, deodorant, inorganic powder and organic fibrous material) described, for example, in JP-A-2003-225565 and JP-A-2006-131767 may be added. As for the additive, a single species thereof may be used alone or two or more species thereof may be used in combination.

The water used for the production of the absorbent resin (C) and the absorbent composition (D) is removed by heating and drying in, for example, a rotary dryer, a puddle dryer, a Nautor-type dryer, or a rotary kiln, whereby the (C) and the (D) in the form of a solid are obtained.

The shapes of the absorbent resin (C) and the absorbent composition (D) of the present invention may be arbitrarily set according to their applications, but in the case of use as a hygienic material such as disposable diaper and sanitary product, a particulate form is preferred. The method for making the (C) and the (D) into a particulate form is not particularly limited, and examples thereof include a method of performing pulverization, particle size adjustment, etc. by a known method.

The method of pulverization is not particularly limited, and an ordinary pulverization apparatus (for example, a hammer pulverizer, an impact pulverizer, a roll pulverizer, and a jet stream pulverizer) or the like may be used. The particle size of the pulverized particles can be adjusted, as necessary, by sieving or the like.

In the case of sieving the particles as needed, the weight average particle diameters of the absorbent resin (C) particles and the absorbent composition (D) particles are, in view of absorption capacity, preferably from 100 to 800 μm, more preferably from 200 to 700 μm, even more preferably from 250 to 600 μm, yet even more preferably from 300 to 500 μm, and most preferably from 350 to 450 μm.

The weight average particle diameter is measured using a Ro-Tap test sieve shaker and standard sieves (JIS Z8801-1:2006) by the method described in Perrys Chemical Engineer's Handbook, 6th edition (McGraw-Hill Book Company, 1984, page 21). Specifically, JIS standard sieves are superposed in order of, beginning from the top, 1,000 μm, 850 μm, 710 μm, 500 μm , 425 μm, 355 μm, 250 μm, 150 μm, 125 μm, 75 μm, and 45 μm, and a receiving tray is placed at the lowermost part. About 50 g of the measurement particle is put on the topmost sieve and shaken for 5 minutes by using the Ro-Tap test sieve shaker. The weight of the measurement particle on each of the sieves and receiving tray is measured, and by taking the total thereof as 100 wt%, the weight fraction of the particle on each sieve is determined. After plotting the obtained value on a logarithmic probability paper [the sieve opening size (particle diameter) as abscissa and the weight fraction as ordinate], a line connecting individual plots is drawn, and a particle diameter corresponding to 50 wt% of the weight fraction is determined. This particle diameter is defined as the weight average particle diameter.

A smaller content of fine particle leads to a higher absorption capacity, and therefore, the content of fine particles of 106 $\mu$m or less (preferably 150 $\mu$m or less) in all particles is preferably 3 wt% or less, more preferably 1 wt% or less. The content of fine particles can be determined using the graph plotting the weight fraction versus the sieve opening size, which is created when determining the weight average particle diameter above.

The shapes of the absorbent resin (C) particle and the absorbent composition (D) particle, which are obtained by the pulverization method above, are not particularly limited, and examples thereof include an indeterminate crushed shape, a scale shape, a pearl shape (a spherical shape), and a rice grain shape. Among these, an indeterminate crushed shape is preferred because good entangling with a fibrous material in an application such as disposable diaper is ensured and the fear of falling off from the fibrous material is eliminated.

The water-retention amounts (g/g) of the absorbent resin (C) particle and the absorbent composition (D) particle of the present invention are preferably from 28 to 45, more preferably from 32 to 40, and particularly preferably from 34 to 38 in view of skin irritation resistance of the absorbent article. The water-retention amount is measured by the following method.

<Measurement Method of Water-Retention Amount>

A measurement sample (1.00g) is put in a teabag (length: 20 cm, width: 10 cm) formed of a nylon net with a mesh-opening of 63 $\mu$m (JIS Z8801-1:2006) and after dipping in 1,000 ml of physiological saline (salt concentration: 0.9 wt%) with no stirring for 1 hour, the teabag is pulled up and hung for 15 minutes to effect draining. Thereafter, the sample with the teabag is put in a centrifuge and centrifugally dehydrated at 150 G for 90 seconds to remove excess physiological saline, and the weight (h1) including the teabag is measured. The weight (h2) of the teabag after the centrifugal dehydration is measured in the same manner as above except not using the measurement sample, and the water-retention amount is determined according to the following formula. Incidentally, the temperatures of the physiological saline used and the measurement atmosphere are 25°C±2°C.

$$\text{Water-retention amount (g/g)} = \text{(h1)} - \text{(h2)}$$

The gel elastic modulus N/m$^2$) of the 30-fold swollen gel obtained by causing 30 parts by weight of an artificial urine to be absorbed into 1 part by weight of the absorbent resin (C) particle or the absorbent composition (D) particle of the present invention is preferably from 2,000 to 3,000, more preferably from 2,025 to 2,950, still more preferably from 2,050 to 2,900, and most preferably from 2,075 to 2,850. Within this range, a further excellent leakage resistance is exhibited on application to an absorbent article. The gel elastic modulus (N/m$^2$) is a value determined by the following measurement method.

<Measurement Method of Gel Elastic Modulus>

In a 100 ml-volume beaker (inner diameter: 5 cm), 60.0 g of artificial urine [a mixture of 200 parts by weight of urea, 80 parts by weight of sodium chloride, 8 parts by weight of magnesium sulfate (7 hydrate), 3 parts by weight of calcium chloride (dihydrate), 2 parts by weight of ferric sulfate (7 hydrate), 9,704 parts by weight of ion-exchange water] was weighed, and in the same manner as the operation described in JIS K7224-1996, 2.0 g of the measurement sample is precisely weighed and charged into the beaker above to prepare a 30-fold swollen gel. The beaker containing the 30-fold swollen gel is allowed to stand in an atmosphere of 40±2°C for 3 hours and further in an atmosphere of 25±2°C for 0. 5 hours, and thereafter, the gel elastic modulus of the 30-fold swollen gel is measured using a curd meter (for example, Curd-Meter MAX ME-500 manufactured by Itec Techno Engineering K.K.). The conditions for measurement with the curd meter are as follows, for example.

- Pressure-sensitive shaft: 8 mm
- Spring: for 100 g
- Load: 100 g
- Elevation rate: 1 inch/7 seconds
- Test property: breakage
- Measurement time: 6 seconds

- Measurement atmosphere temperature: $25\pm2°C$

The gel elastic modulus is further enhanced by applying a crosslinking treatment to the surfaces of the absorbent resin (C) particle and the absorbent composition (D) particle by using a crosslinking agent.

Examples of the crosslinking agent used for the surface crosslinking treatment (hereinafter, sometimes referred to as surface crosslinking agent) include the (k1) polyhydric alcohol having 2 to 6 carbon atoms, the (k2) polyvalent glycidyl ether having 8 to 21 carbon atoms, the (k3) polyvalent amine having 2 to 6 carbon atoms, the (k4) alkanolamine having 2 to 8 carbon atoms, the (k5) polyvalent aziridine compound having 6 to 12 carbon atoms, polyvalent organic isocyanate compounds described in JP-A-59-189103 , silane coupling agents described in JP-A-61-211305 and JP-A-61-252212, and polyvalent metals described in JP-A-51-136588 and JP-A-61-257235. In this connection, the crosslinking agent for obtaining the (A2) and (B2) is hereinafter sometimes referred to as an internal crosslinking agent.

Among these, for example, (k2), (k3) and the silane coupling agents are preferred, (k2) and the silane coupling agents are more preferred, and (k2) is particularly preferred in view of absorption performance.

In the case of performing the surface crosslinking treatment, the amount of the crosslinking agent used is preferably from 0.001 to 3 wt% based on the weight of the absorbent resin (C) or absorbent composition (D) before surface crosslinking, more preferably from 0.005 to 2 wt%, and particularly preferably from 0.01 to 1 wt% in view of absorption performance, biodegradability, etc.

The surface crosslinking treatment can be performed by spraying a mixed solution of the surface crosslinking agent above, water and a solvent onto the surface of the absorbent resin (C) or the absorbent composition (D) particle by a known method and allowing a reaction to proceed by heating. The reaction temperature is usually from 100 to 230°C, preferably from 120 to 160°C. The reaction time may be varied according to the reaction temperature but is usually from 3 to 60 minutes, preferably from 10 to 40 minutes. The particulate absorbent resin and/or the absorbent composition obtained by thus performing surface crosslinking may further be surface-crosslinked by a different crosslinking agent.

By incorporating (E) a hydrophobic substance into the absorbent resin (C) particle and the absorbent composition (D) particle, the absorption velocity pattern can be controlled (the velocity is slow in the initial stage, moderate in the middle stage, and fast in the late stage) and the leakage resistance is enhanced as described in JP-A-2011-252088, so that there can be obtained (P-1) an absorbent resin particle and (P-2) an absorbent composition particle each suitable for an absorbent article (e.g., a hygienic material such as disposable diaper and sanitary product) free from a problem of skin irritation.

The hydrophobic substance (E) is a compound having at least one monovalent aliphatic hydrocarbon group with 8 to 26 carbon atoms and having at least one functional group capable of forming a hydrogen bond with a carboxyl group and is present in a specific amount in the inside of the absorbent resin particle (P-1) and the absorbent composition particle (P-2) and present in a specific amount on the surface of the (P-1) and the (P-2). This is described infra.

Examples of the monovalent aliphatic hydrocarbon group with 8 to 26 carbon atoms include an octyl group, a decyl group, a dodecyl group, a tetradecyl group, a hexadecyl group, an octadecyl group, an eicosyl group, a docosyl group, a tetracosyl group, and a hexacosyl group.

Examples of the functional group capable of forming a hydrogen bond with a carboxyl group include a carboxyl group (including a salt thereof), a phosphoric acid group (including a salt thereof), a sulfo group (including a salt thereof), a primary amino group (including a salt thereof), a secondary amino group (including a salt thereof), a tertiary amino group (including a salt thereof), a hydroxyl group, an oxycarbonyl group, a phosphoric acid ester group, a sulfonic acid ester group, an amide group, a urethane group, and a urea group.

In a single molecule of the hydrophobic substance (E), the number of monovalent aliphatic hydrocarbon groups with 8 to 26 carbon atoms is, in view of skin irritation resistance of the absorbent article, preferably from 1 to 5, more preferably from 1 to 4, particularly preferably from 1 to 3. In a single molecule of (E), the number of functional groups capable of forming a hydrogen bond with a carboxyl group is, in view of leakage resistance of the absorbent article, preferably from 1 to 5, more preferably from 1 to 4, particularly preferably from 1 to 3.

Examples of the hydrophobic substance (E1) having a carboxyl group include a nonanoic acid, a dodecanoic acid, an octadecanoic acid, and a heptacosanoic acid. Examples of the salt thereof include salts with sodium, potassium, zinc, calcium, magnesium and aluminum (hereinafter, simply referred to as Na, K, Zn, Ca, Mg and Al).

Examples of the hydrophobic substance (E2) having a phosphoric acid group include an octylphosphoric acid, an octadecylphosphoric acid, and a hexacosylphosphoric acid. Examples of the salt thereof include salts with Na, K, Zn, Ca, Mg and Al.

Examples of the hydrophobic substance (E3) having a sulfonic acid group include an octylsulfonic acid, an octadecylsulfonic acid, and a hexacosylsulfonic acid. Examples of the salt thereof include salts with Na, K, Zn, Ca, Mg and Al.

Examples of the hydrophobic substance (E4) having a primary amino group include octylamine, octadecylamine, and hexacosylamine. Examples of the salt thereof include salts with hydrochloric acid, carboxylic acid, sulfuric acid and nitric acid.

Examples of the hydrophobic substance (E5) having a secondary amino group include methyloctylamine, methylhexacosylamine, octylhexacosylamine, dihexaccsylamine, and methyloctadecylamine. Examples of the salt thereof include salts with hydrochloric acid, carboxylic acid, sulfuric acid and nitric acid.

Examples of the hydrophobic substance (E6) having a tertiary amino group include dimethyloctylamine, dimethylhexacosylamine, methyloctylhexacosylamine, and methyldihexacosylamine. Examples of the salt thereof include salts with hydrochloric acid, carboxylic acid, sulfuric acid and nitric acid.

Examples of the hydrophobic substance (E7) having a hydroxyl group include octyl alcohol, octadecyl alcohol, and hexacosyl alcohol.

Examples of the hydrophobic substance (E8) having an oxycarbonyl group include an esterification product of a long-chain fatty acid with 8 to 26 carbon atoms and an alcohol with 1 to 26 carbon atoms having at least one hydroxyl group, and an esterification product of a long-chain aliphatic alcohol with 8 to 26 carbon atoms and a carboxylic acid having a hydrocarbon group with 1 to 7 carbon atoms and having at least one carboxyl group.

Examples of the esterification product of a long-chain fatty acid with 8 to 26 carbon atoms and an alcohol with 1 to 26 carbon atoms having at least one hydroxyl group include methyl nonanoate, methyl heptacosanoate, hexacosyl nonanoate, hexacosyl heptacosanoate, octyl octadecanoate, glycerin monononanoate, glycerin monooctadecanoate, glycerin monoheptacosanoate, pentaerythritol monononanoate, pentaerythritol monooctadecanoate, pentaerythritol monoheptacosanoate, sorbitol monononanoate, sorbitol monooctadecanoate, sorbitol monoheptacosanoate, sucrose monononanoate, sucrose dinonanoate, sucrose trinonanoate, sucrose monooctadecanoate, sucrose dioctadecanoate, sucrose trioctadecanoate, sucrose monoheptacosanoate, sucrose diheptacosanoate, sucrose triheptacosanoate, and beef tallow.

Examples of the esterification product of a long-chain aliphatic alcohol with 8 to 26 carbon atoms and a carboxylic acid with 1 to 8 carbon atoms having at least one carboxyl group include octyl nonanoate, octyl acetate, octyl octylate, hexacosyl acetate, and hexacosyl octylate.

The hydrophobic substance (E9) having a phosphoric acid ester group includes a dehydrated condensate of a long-chain aliphatic alcohol with 8 to 26 carbon atoms and a phosphoric acid, and a salt thereof. Examples thereof include octyl phosphate, octadecyl phosphate, and hexacosyl phosphate. Examples of the salt thereof include salts with Na and K. The phosphoric acid ester includes diester and triester as well as monoester.

The hydrophobic substance (E10) having a sulfuric acid ester group includes a dehydrated condensate of a long-chain aliphatic alcohol with 8 to 26 carbon atoms and a sulfuric acid, and a salt thereof. Examples thereof include octyl sulfate, octadecyl sulfate, and hexacosyl sulfate. Examples of the salt thereof include salts with Na and K. The sulfuric acid ester includes diester as well as monoester.

The hydrophobic substance (E11) having an amide group includes an amidation product of a long-chain aliphatic primary amine with 8 to 26 carbon atoms and a carboxylic acid having a hydrocarbon group with 1 to 26 carbon atoms, an amidation product of ammonia or a primary amine with 1 to 7 carbon atoms and a long-chain fatty acid with 8 to 26 carbon atoms, an amidation product of a long-chain aliphatic secondary amine having at least one aliphatic chain with 8 to 26 carbon atoms and a carboxylic acid with 1 to 26 carbon atoms, and an amidation product of a secondary amine having two aliphatic hydrocarbon groups with 1 to 7 carbon atoms and a long-chain fatty acid with 8 to 26 carbon atoms.

The amidation product of a long-chain aliphatic primary amine with 8 to 26 carbon atoms and a carboxylic acid having a hydrocarbon group with 1 to 26 carbon atoms includes those obtained by reacting a primary amine and a carboxylic acid at 1 : 1 and at 1 : 2. Examples of those obtained by the reaction at 1 : 1 include acetic acid N-octylamide, acetic acid N-hexacosylamide, heptacosanoic acid N-octylamide, and heptacosanoic acid N-hexacosylamide. Examples of those obtained by the reaction at 1 : 2 include diacetic acid N-octylamide, diacetic acid N-hexacosylamide, diheptacosanoic acid N-octylamide, and diheptacosanoic acid N-hexacosylamide. In the case of reacting a primary amine and a carboxylic acid at 1 : 2, the carboxylic acids used may be either the same or different.

The amidation products of ammonia or a primary amine with 1 to 7 carbon atoms and a long-chain fatty acid with 8 to 26 carbon atoms are classified into those obtained by the reaction of ammonia or a primary amine with a carboxylic acid at 1 : 1 and those obtained by the reaction at 1 : 2. Examples those obtained by the reaction at 1 : 1 include nonanoic acid amide, nonanoic acid methylamide, nonanoic acid N-heptylamide, heptacosanoic acid amide, heptacosanoic acid N-methylamide, heptacosanoic acid N-heptylamide, and heptacosanoic acid N-hexacosylamide. Examples of those obtained by the reaction at 1 : 2 include dinonanoic acid amide, dinonanoic acid N-methylamide, dinonanoic acid N-heptylamide, diheptacosanoic acid amide, diheptacosanoic acid N-methylamide, diheptacosanoic acid N-heptylamide, and diheptacosanoic acid N-hexacosylamide. In the case of reacting ammonia or a primary amine with a carboxylic acid at 1 : 2, the carboxylic acids used may be either the same or different.

Examples of the amidation product of a long-chain aliphatic secondary amine having at least one aliphatic chain with 8 to 26 carbon atoms and a carboxylic acid with 1 to 26 carbon atoms include acetic acid N-methyloctylamide, acetic acid N-methylhexacosylamide, acetic acid N-octylhexacosylamide, acetic acid N-dihexacosylamide, heptacosanoic acid N-methyloctylamide, heptacosanoic acid N-methylhexacosylamide, heptacosanoic acid N-octylhexacosylamide, and heptacosanoic acid N-dihexacosylamide.

Examples of the amidation product of a secondary amine having two aliphatic hydrocarbon groups with 1 to 7 carbon atoms and a long-chain fatty acid with 8 to 26 carbon atoms include nonanoic acid N-dimethylamide, nonanoic acid N-methylheptylamide, nonanoic acid N-diheptylamide, heptacosanoic acid N-dimethylamide, heptacosanoic acid N-methylheptylamide, and heptacosanoic acid N-diheptylamide.

The hydrophobic substance (E12) having a urethane group includes a reaction product of a long-chain aliphatic alcohol with 8 to 26 carbon atoms and a compound having at least one isocyanate group. Examples of the long-chain aliphatic alcohol include octyl alcohol, octadecyl alcohol, and hexacosyl alcohol, and examples of the compound having at least one isocyanate group include methyl isocyanate, dodecyl isocyanate, hexacosyl isocyanate, methylene diisocyanate, and hexamethylene diisocyanate.

The hydrophobic substance (E13) having a urea group includes a reaction product of a primary or secondary amine having a hydrocarbon group with 8 to 26 carbon atoms and a compound having at least one isocyanate group. Examples of the primary amine include octylamine, octadecylamine, and hexacosylamine. Examples of the secondary amine include methyloctylamine, methylhexacosylamine, octylhexacosylamine, and dihexacosylamine. Examples of the compound having at least one isocyanate group include methyl isocyanate, dodecyl isocyanate, hexacosyl isocyanate, methylene diisocyanate, and hexamethylene diisocyanate.

Among these hydrophobic substances (E), in view of leakage resistance of the absorbent article, a compound having, as the functional group capable of forming a hydrogen bond with a carboxyl group, at least one member selected from the group consisting of a carboxyl group (including a salt thereof), a phosphoric acid group (including a salt thereof), a sulfo group (including a salt thereof), a primary amino group (including a salt thereof), a secondary amino group (including a salt thereof), a tertiary amino group (including a salt thereof), a hydroxyl group, an oxycarbonyl group, a phosphoric acid ester group, a sulfonic acid ester group and an amide group is preferred, and a compound having at least one member selected from the group consisting of a carboxyl group (including a salt thereof), a primary amino group (including a salt thereof), a secondary amino group (including a salt thereof), a tertiary amino group (including a salt thereof), a hydroxyl group, an oxycarbonyl group and an amide group is more preferred. As for the hydrophobic substance (E), a single species thereof may be used alone or two or more species thereof may be used in combination.

As described above, the hydrophobic substance (E) is present in the following specific amount in the inside of the absorbent resin particle (P-1) and the absorbent composition particle (P-2) and present in the following specific amount on the surface of the (P-1) and the (P-2).

In view of skin irritation resistance and leakage resistance in use for an absorbent article, the content of the hydrophobic substance (E) in the inside of the (P-1) and the (P-2) is preferably from 0.01 to 10.0 wt%, more preferably from 0.01 to 5.0 wt%, still more preferably from 0.05 to 2.0 wt%, and most preferably from 0.1 to 1.0 wt%, based on the weight of the (P-1) or the (P-2), respectively.

In view of skin irritation resistance and leakage resistance in use for an absorbent article, the content of the hydrophobic substance (E) present on the surface of the (P-1) and the (P-2) is from 0.001 to 1.0 wt%, preferably from 0.005 to 0.5 wt%, more preferably from 0.01 to 0.3 wt%, still more preferably from 0.01 to 0.1 wt%, based on the weight of the (P-1) or the (P-2), respectively.

The content of the hydrophobic substance (E) present on the surface is measured by the following method. The content of the hydrophobic substance (E) present in the inside is calculated from the amount obtained by subtracting the amount of the hydrophobic substance (E) present on the surface from the amount of the hydrophobic substance (E) used for the production.

<Measurement Method of Content of Hydrophobic Substance (E) >

One part by weight of the absorbent resin particle (P-1) or the absorbent composition particle (P-2) and 1,000 parts by weight of an organic solvent (an organic solvent capable of dissolving at least 0.01 parts by weight of the hydrophobic substance per 100 parts by weight of the organic solvent at from 25 to 110°C; here, the temperature allowing for this dissolution is referred to as dissolution temperature) are added to a glass beaker and left standing at the dissolution temperature for 24 hours to obtain an extraction solution of the hydrophobic substance. The extraction solution is filtrated using a filter paper and collected into a glass beaker weighed in advance, and after evaporating the solvent, the beaker is weighed. The numerical value obtained by subtracting the weight of the beaker weighed in advance from the weight after evaporation of the filtrate is multiplied by 100. Using the sample after extraction remaining on the filter paper, the same operation is repeated twice, and the total amount of the evaporation-to-dryness matters obtained by three-time extraction is taken as the content (wt%) of the hydrophobic substance present on the surface.

In the method of measuring a swollen volume per 1 g of the absorbent resin particle of the present invention for physiological saline, the ratio (t2/t1) of the time (t1) taken until the swollen volume reaches 5 ml and the time (t2) taken until the swollen volume reaches 40 ml is preferably from 5 to 20, more preferably from 5 to 15, and most preferably from 5 to 10. Besides, t1 is preferably from 20 to 60 seconds, more preferably from 20 to 50 seconds, and most preferably from 30 to 40 seconds. Within this range, the skin irritation resistance of an absorbent article is further improved.

The swollen volume is measured by the following method.

<Measuring Apparatus>

The method of measuring a swollen volume is a measurement method performed in a room at $25\pm2°C$ and a humidity of $50\pm10\%$ typically by using the apparatus shown in Fig. 1. In this connection, the temperature of the physiological saline used is $25\pm2°C$. The apparatus shown in Fig. 1 is composed of an acrylic bottomed cylinder 1 and an acrylic disk 2. Incidentally, numerical values relevant to the apparatus in the following description are examples, and the present invention is not limited to these numerical values. The numerical values relevant to the measurement method are also examples.

The bottomed cylinder 1 is a bottomed cylinder having a bottom at one opening of a cylinder with an inner diameter of 81 mm and a length of 35 mm and being opened at the other side. The acrylic disk 2 is a disk with an outer diameter of 80.5 mm and a thickness of 12 mm. The disk 2 has a circular concave with a diameter of 70.5 mm and a depth of 4 mm at a position where the center of the disk is coincident with the center of the circle. The disk 2 has, as a handgrip, a column with a length of 13 mm and an outer diameter of 15 mm in the circular concave portion, at a position where the center of the disk 2 is coincident with the center of bottom face of the column. Furthermore, in the disk 2, 64 holes with a diameter of 2 mm are perforated in a radial pattern. The holes of the disk 2 are described below. The holes are perforated such that 5 holes with a diameter of 2 mm are present on each of lines dividing the disk into equal eight portions and are located at equal intervals of 5 mm between a position of 10 mm and a position of 30 mm from the center of the disk (40 holes in total). In addition, 3 holes with a diameter of 2 mm are present on each of lines dividing the disk into equal eight portions and being tilted at 22.5° from the above-described equally dividing lines and are located at equal intervals of 5 mm between a position of 20 mm and a position of 30 mm from the center of the disk (24 holes in total). The weight of the disk 2 is $60\pm5$ g.

<Measurement Procedure of Swollen Volume>

Into the bottom plate-attached cylinder 1 erected vertically, 2.50 g of a measurement sample sieved to a particle diameter of 150 to 850 $\mu$m (water content: 8.0% or less) is weighed and charged to lie at an almost uniform thickness on the bottom of the bottom plate-attached cylinder 1. The disk 2 is placed thereon with the columrar handgrip up, and the distance from the bottom face of the cylinder to the top face of the handgrip of the disk is measured using a thickness meter (for example, Digimatic Indicator ID-F150, manufactured by Mitutoyo Co.). At this time, the pressure imposed on the measurement sample due to the weight of the measuring rod ($140\pm10$ g) of the Digimatic Indicator and the weight of the disk 2 with the handgrip is $3.9\pm0.3$ g/cm$^2$. Then, the thickness indication of the Digimatic Indicator is set to zero. Subsequently, 120 ml of physiological saline is charged into the bottomed cylinder 1 within 2 seconds. The time of the start of charging is taken as zero, and the distance H (cm) for which the disk 2 is elevated with the passage of time from the start of charging is recorded as continuous data. The swollen volume (ml) per 1 g of the measurement sample is calculated according to the following formula, whereby data of change in swollen volume with respect to the time are obtained. From the data, the time (t1) taken until the swollen volume reaches 5 ml and the time (t2) taken until the swollen volume reaches 40 ml are determined. The measurement is performed five times, and the average value thereof is used as the measured value.

[Math. 1]

$$\text{Swollen volume (ml/g)} \; = \; \frac{\text{bottom area (cm}^2\text{) within bottom plate-attached cylinder} \times H \text{ (cm)}}{\text{weight (g) of measurement sample}}$$

Examples of the method for incorporating the hydrophobic substance (E) into the absorbent resin (C) particle and the absorbent composition (D) particle to produce the absorbent resin particle (P-1) and the absorbent composition particle (P-2) include (1) a method of mixing/kneading the hydrophobic substance (E) and a hydrous gel of the (C) or the (D) at preferably from 20 to 100°C, more preferably from 40 to 90°C, still more preferably from 50 to 80°C, and then forming the mixture into particles, and (2) a method of producing (B1) and/or (B2) in the presence of the hydrophobic substance (E) and (A1) and/or (A2), and then forming the product into particles.

In the production process of the absorbent resin (C) and the absorbent composition (D), the timing of binding or mixing the (A1) and/or the (A2) with the (B1) and/or the (B2) is described below by referring to the following general steps [1] to [4] relevant to the (B1) and/or the (B2):

[1] a step of subjecting a (meth)acrylic acid monomer and/or its salt obtained by neutralization with a neutralizing agent to radial polymerization to produce (B1) and/or (B2),

[2] a step of neutralizing the (B1) and/or the (B2),

[3] a step of drying the (B1) and/or the (B2), and

[4] a step of surface-crosslinking the (B1) and/or the (B2).

In step [1], the (B1) and/or the (B2) is produced in the presence of the (A1) and/or the (A2), whereby an absorbent resin (C) in which the (B1) and/or the (B2) are grafted to the (A1) and/or the (A2) is obtained.

A step of binding the (A1) and/or the (A2) to the (B1) and/or the (B2) with a binder is provided between step [1] and step [2] or between step [2] and step [3], whereby an absorbent resin (C) is obtained.

In step [3], a binding reaction of the (A1) and/or the (A2) to the (B1) and/or the (B2) is performed in the course of drying by allowing the (A1) and/or the (A2) and a binder to be present together, whereby the absorbent resin (C) is obtained.

In the absorbent composition (D), mixing of the (A1) and/or the (A2), the (B1) and/or the (B2), and the absorbent resin (C) that is used if desired, may be performed at arbitrary timing as long as it is after the completion of step [1]. However, when mixed after the completion of step [3], since the (B1) and/or the (B2) are a solid, the absorbent composition (D) is obtained in the form of a powder blend. The (A1) and/or the (A2) used after the completion of step [3] may have been subjected to a surface crosslinking treatment. Furthermore, when a mixture of the (A1) and/or the (A2) with the (B1) and/or the (B2) is surface-crosslinked in step [4], the absorbent composition (D) can be obtained as a composition having the absorbent resin (C) only on the surface.

The absorbent resin, the absorbent resin particle, the absorbent composition, and the absorbent composition particle of the present invention can be individually or in combination used together with at least one member selected from the group consisting of a fiber, a nonwoven fabric and a woven fabric to produce an absorbent material.

Examples of the fiber, the nonwoven fabric, or the woven fabric include various fluff pulps, cotton-like pulps, and fibrous materials conventionally used for an absorbent article [the feedstock (e.g., softwood, hardwood), the production method {e.g., chemical pulp, semichemical pulp, chemithermomechanical pulp (CTMP)}, the bleaching method, etc. are not particularly limited]. In addition to these fibrous materials, a synthetic fiber that is not swellable with water may also be used alone or in combination with the above-described fluff pulp, cotton-like pulp or the like. Examples of the synthetic fiber include a polyolefin-based fiber (e.g., polyethylene-based fiber and polypropylene-based fiber), a polyester-based fiber (e.g., polyethylene terephthalate fiber), a polyolefin/polyester composite fiber, a polyamide fiber, and a polyacrylonitrile fiber. The structure, production method, etc. of the absorbent material are those conventionally known (e.g., JP-A-2003-225565).

This absorbent material is suitably used for an absorbent article [e. g. , disposable diaper, sanitary napkin, paper towel, pad (e.g., incontinence pad, surgical underpad) and sheet for pet animals]. The production method, etc. of the absorbent article are those conventionally known (for example, JP-A-2003-225565).

In the case of using the absorbent resin, the absorbent resin particle, the absorbent composition, and the absorbent composition particle of the present invention together with at least one member selected from the group consisting of a fiber, a nonwoven fabric and a woven fabric to produce an absorbent material, the weight ratio thereof to the fiber or the like (the weight of the absorbent resin particle or the like/the weight of fiber or the like) is preferably from 40/60 to 70/30, more preferably from 50/50 to 60/40.

EXAMPLES

The present invention is further described below by reference to examples and comparative examples, but the present invention is not limited thereto. In the following, "parts" indicates "parts by weight".

<Production Example 1> Production of Hydrous Gel of Crosslinked Polyacrylic Acid:

155 Parts (2.15 molar parts) of acrylic acid (produced by Mitsubishi Chemical Corporation, purity: 100%), 0.6225 parts (0.0024 molar parts) of pentaerythritol triallyl ether (produced by Daiso Co. , Ltd.) as an internal crosslinking agent, and 340.27 parts of deionized water were kept at 3°C with stirring/mixing. After flowing nitrogen into the mixture to make a dissolved oxygen concentration of 1 ppm or less, 0.62 parts of an aqueous 1% hydrogen peroxide solution, 1.1625 parts of an aqueous 2% ascorbic acid solution, 2.325 parts of an aqueous 2% 2,2'-azobis[2-methyl-N-(2-hydroxyethyl)-propionamide] solution, and 1.9 parts of Surfactant (A-1) (hexadecanoic acid ethylene oxide 2-mol adduct) were added/mixed to initiate polymerization. After the temperature of the mixture reached 90°C, polymerization was allowed to proceed at $90\pm2°C$ for about 5 hours, whereby 502.27 parts by weight of Hydrous Gel (B2-1) of a crosslinked polyacrylic acid was obtained.

<Production Example 2> Production of Aqueous Solution of Linear Polyacrylic Acid:

501.65 Parts by weight of Aqueous Solution (B1-1) of a linear polyacrylic acid was obtained in the same manner as in Production Example 1 except not adding pentaerythritol triallyl ether as an internal crosslinking agent.

<Production Example 3> Production of Aqueous Solution (A1-1) of Oxidized Polysaccharide:

5 Parts of starch (MS-3800, produced by Nihon Shokuhin Kako Co. , Ltd.) and 100 parts of ion-exchanged water were added and stirred. 10 Parts of potassium permanganate was dissolved in 500 parts of ion-exchanged water, and the resulting solution was added over 6 hours. During the addition, pH was kept at 12 by adding an aqueous 2 N sodium hydroxide (NaOH) solution. After the completion of the addition of potassium permanganate, stirring was further continued for 2 hours, and the unreacted potassium permanganate was reduced with sodium sulfite. Manganese dioxide as a by-product was separated by filtration, and the filtrate was purified by performing diffusion dialysis with ion-exchanged water for 5 days and then concentrated to obtain Aqueous Solution (A1-1) of 30 wt% oxidized polysaccharide. The Mw of the obtained oxidized polysaccharide was 6,000, and the acid value was 300 mgKOH/g.

<Production Example 4> Production of Aqueous Solution (A1-2) of Oxidized Polysaccharide:

Aqueous Solution (A1-2) of 30 wt% oxidized polysaccharide was obtained in the same manner as in Production Example 3 except using corn starch (produced by Wako Pure Chemical Industries, Ltd.) in place of starch and changing the charge amount of potassium permanganate to 18 parts and the addition time to 2 hours. The Mw of the obtained oxidized polysaccharide was 900,000, and the acid value was 524 mgKOH/g.

<Production Example 5> Production of Aqueous Solution (A1-3) of Oxidized Polysaccharide:

Aqueous Solution (A1-3) of 30 wt% oxidized polysaccharide was obtained in the same manner as in Production Example 3 except changing the charge amount of potassium permanganate to 2.0 parts and the addition time to 1 hour. The Mw of the obtained oxidized polysaccharide was 100,000, and the acid value was 66 mgKOH/g.

<Production Example 6> Production of Aqueous Solution (A1-4) of Oxidized Polysaccharide:

Aqueous Solution (A1-4) of 30 wt% oxidized polysaccharide was obtained in the same manner as in Production Example 3 except using Kiprogum (M-800A, produced by Nippon Starch Chemical Co. , Ltd.) in place of starch. The Mw of the obtained oxidized polysaccharide was 85,000, and the acid value was 280 mgKOH/g.

<Production Example 7> Production of Aqueous Solution (A1-5) of Oxidized polysaccharide:

While stirring 31.9 parts of an aqueous 12 wt% sodium hydroxide solution, 5 parts of cellulose (KC FLOCK W-400G, produced by Nippon Paper Chemicals Co. , Ltd.) was added thereto and uniformly mixed at 25°C for 90 minutes to mercerize the cellulose. 13.0 Parts of potassium permanganate was dissolved in 200 parts of ion-exchanged water, and the resulting solution was added for 10 hours. During the addition, pH was kept at 12 by adding an aqueous 2 mol/L sodium hydroxide solution. The subsequent procedure was performed in the same manner as in Production Example 3 to obtain Aqueous Solution (A1-5) of 30 wt% oxidized polysaccharide. The Mw of the obtained oxidized polysaccharide was 320, 000, and the acid value was 400 mgKOH/g.

<Production Example 8> Production of Aqueous Solution (A1-6) of Oxidized Polysaccharide:

Aqueous Solution (A1-6) of 30 wt% oxidized polysaccharide was obtained in the same manner as in Production Example 3 except using 6. 3 parts of carboxymethyl cellulose (produced by Aldrich Chemical Co. Inc., substitution degree: 0.7) in place of 5 parts of starch. The Mw of the obtained oxidized polysaccharide was 300,000, and the acid value was 500 mgKOH/g.

<Production Example 9> Production of Hydrous Gel (A2-1) of Crosslinked Product of Oxidized Polysaccharide:

9.7 Parts of an aqueous 48.5 wt% sodium hydroxide solution and 0.5 parts of a 2 wt% water/methanol mixed solution (weight ratio of water/methanol = 70/30) of ethylene glycol diglycidyl ether were added per 100 parts of Aqueous Solution (A1-1) of 30 wt% oxidized polysaccharide produced in Production Example 3, and the mixture was stirred at 90°C for 30 minutes to obtain 110.2 parts of Hydrous Gel (A2-1) of a crosslinked product of an oxidized polysaccharide.

<Production Example 10> Production of Hydrous Gel (A2-2) of Crosslinked Product of Oxidized Polysaccharide:

115.5 Parts of Hydrous Gel (A2-2) of a crosslinked product of an oxidized polysaccharide was obtained in the same manner as in Production Example 9 except using Aqueous Solution (A1-2) of an oxidized polysaccharide produced in Production Example 4 in place of Aqueous Solution (A1-1) of an oxidized polysaccharide and changing the charge amount of an aqueous 48.5 wt% sodium hydroxide solution to 16.2 parts.

<Production Example 11> Production of Hydrous Gel (A2-3) of Crosslinked Product of Oxidized Polysaccharide:

102.1 Parts of Hydrous Gel (A2-3) of a crosslinked product of an oxidized polysaccharide was obtained in the same manner as in Production Example 9 except using Aqueous Solution (A1-3) of an oxidized polysaccharide produced in Production Example 5 in place of Aqueous Solution (A1-1) of an oxidized polysaccharide and changing the charge amount of an aqueous 48.5 wt% sodium hydroxide solution to 2.1 parts.

<Production Example 12> Production of Hydrous Gel (A2-4) of Crosslinked Product of Oxidized Polysaccharide:

101.9 Parts of Hydrous Gel (A2-4) of a crosslinked product of an oxidized polysaccharide was obtained in the same manner as in Production Example 9 except using Aqueous Solution (A1-4) of an oxidized polysaccharide produced in Production Example 6 in place of Aqueous Solution (A1-1) of an oxidized polysaccharide and changing the charge amount of an aqueous 48.5 wt% sodium hydroxide solution to 2.1 parts.

<Production Example 13> Production of Hydrous Gel (A2-5) of Crosslinked Product of Oxidized Polysaccharide:

108.1 Parts of Hydrous Gel (A2-5) of a crosslinked product of an oxidized polysaccharide was obtained in the same manner as in Production Example 9 except using Aqueous Solution (A1-5) of an oxidized polysaccharide produced in Production Example 7 in place of Aqueous Solution (A1-1) of an oxidized polysaccharide and changing the charge amount of an aqueous 48.5 wt% sodium hydroxide solution to 8.7 parts.

<Production Example 14> Production of Hydrous Gel (A2-6) of Crosslinked Product of Oxidized Polysaccharide:

116.0 Parts of Hydrous Gel (A2-6) of a crosslinked product of an oxidized polysaccharide was obtained in the same manner as in Production Example 9 except using Aqueous Solution (A1-6) of an oxidized polysaccharide produced in Production Example 8 in place of Aqueous Solution (A1-1) of an oxidized polysaccharide and changing the charge amount of an aqueous 48.5 wt% sodium hydroxide solution to 12.3 parts.

<Example 1> Production of Hydrous Gel (G1) of Graft Polymer:

93 Parts (1.29 molar parts) of acrylic acid (produced by Mitsubishi Chemical Corporation, purity: 100%), 0.3735 parts (0.0014 molar parts) of pentaerythritol triallyl ether (produced by Daiso Co. , Ltd.) as an internal crosslinking agent, 2.475 parts of a 2 wt% water/methanol mixed solution (weight ratio of water/methanol = 70/30) of ethylene glycol diglycidyl ether, and 340.27 parts of deionized water in which 206.7 parts of Aqueous Solution (A1-1) of an oxidized polysaccharide produced by Production Example 3 was previously dissolved were kept at 3°C with stirring/mixing. After flowing nitrogen into the mixture to make a dissolved oxygen concentration of 1 ppm or less, 0. 372 parts of an aqueous 1% hydrogen peroxide solution, 0.698 parts of an aqueous 2% ascorbic acid solution, and 1.395 parts of an aqueous 2% 2,2'-azobis[2-methyl-N-(2-hydroxyethyl)-propionamide] solution were added/mixed to initiate polymerization. After the temperature of the mixture reached 90°C, polymerization was allowed to proceed at $90 \pm 2$°C for about 5 hours, whereby 501.01 parts of Hydrous Gel (G1) of a graft polymer was obtained.

<Example 2> Production of Hydrous Gel (G2) of Graft Polymer:

Hydrous Gel (G2) of a graft polymer was obtained in the same manner as in Example 1 except using Aqueous Solution (A1-2) of an oxidized polysaccharide in place of Aqueous Solution (A1-1) of an oxidized polysaccharide.

<Example 3> Production of Hydrous Gel (G3) of Graft Polymer:

Hydrous Gel (G3) of a graft polymer was obtained in the same manner as in Example 1 except changing the charge amount of acrylic acid to 108.5 parts and using 155.5 parts of Aqueous Solution (A1-3) of an oxidized polysaccharide in place of 206.7 parts of Aqueous Solution (A1-1) of an oxidized polysaccharide.

<Example 4> Production of Hydrous Gel (G4) of Graft Polymer:

Hydrous Gel (G4) of a graft polymer was obtained in the same manner as in Example 1 except using Aqueous Solution (A1-3) of an oxidized polysaccharide in place of Aqueous Solution (A1-1) of an oxidized polysaccharide.

<Example 5> Production of Hydrous Gel (G5) of Graft Polymer:

Hydrous Gel (G5) of a graft polymer was obtained in the same manner as in Example 1 except changing the charge amount of acrylic acid to 31 parts and using 413.3 parts of Aqueous Solution (A1-3) of an oxidized polysaccharide in place of 206.7 parts of Aqueous Solution (A1-1) of an oxidized polysaccharide.

<Example 6> Production of Hydrous Gel (G6) of Graft Polymer:

Hydrous Gel (G6) of a graft polymer was obtained in the same manner as in Example 1 except using Aqueous Solution (A1-4) of an oxidized polysaccharide in place of Aqueous Solution (A1-1) of an oxidized polysaccharide.

<Example 7> Production of Hydrous Gel (G7) of Graft Polymer:

Hydrous Gel (G7) of a graft polymer was obtained in the same manner as in Example 1 except using Aqueous Solution (A1-5) of an oxidized polysaccharide in place of Aqueous Solution (A1-1) of an oxidized polysaccharide.

<Example 8> Production of Hydrous Gel (G8) of Graft Polymer:

Hydrous Gel (G8) of a graft polymer was obtained in the same manner as in Example 1 except changing the charge amount of acrylic acid to 108.5 parts and using 155.5 parts of Aqueous Solution (A1-6) of an oxidized polysaccharide in place of 206.7 parts of Aqueous Solution (A1-1) of an oxidized polysaccharide.

<Example 9> Production of Hydrous Gel (G9) of Graft Polymer:

Hydrous Gel (G9) of a graft polymer was obtained in the same manner as in Example 1 except using Aqueous Solution (A1-6) of an oxidized polysaccharide in place of Aqueous Solution (A1-1) of an oxidized polysaccharide.

<Example 10> Production of Hydrous Gel (G10) of Graft Polymer:

Hydrous Gel (G10) of a graft polymer was obtained in the same manner as in Example 1 except changing the charge amount of acrylic acid to 31 parts and using 413.3 parts of Aqueous Solution (A1-6) of an oxidized polysaccharide in place of 206.7 parts of Aqueous Solution (A1-1) of an oxidized polysaccharide.

<Example 11> Production of Hydrous Gel (G11) of Graft Polymer:

Hydrous Gel (G11) of a graft polymer was obtained in the same manner as in Example 1 except not adding pentaerythritol triallyl ether (produced by Daiso Co., Ltd.) as an internal crosslinking agent.

<Example 12> Production of Hydrous Gel (G12) of Graft Polymer:

Hydrous Gel (G12) of a graft polymer was obtained in the same manner as in Example 11 except using Aqueous Solution (A1-2) of an oxidized polysaccharide in place of Aqueous Solution (A1-1) of an oxidized polysaccharide.

<Example 13> Production of Hydrous Gel (G13) of Graft Polymer:

Hydrous Gel (G13) of a graft polymer was obtained in the same manner as in Example 11 except changing the charge amount of acrylic acid to 108.5 parts and using 155.5 parts of Aqueous Solution (A1-3) of an oxidized polysaccharide in place of 206.7 parts of Aqueous Solution (A1-1) of an oxidized polysaccharide.

<Example 14> Production of Hydrous Gel (G14) of Graft Polymer:

Hydrous Gel (G14) of a graft polymer was obtained in the same manner as in Example 11 except using Aqueous Solution (A1-3) of an oxidized polysaccharide in place of Aqueous Solution (A1-1) of an oxidized polysaccharide.

<Example 15> Production of Hydrous Gel (G15) of Graft Polymer:

Hydrous Gel (G15) of a graft polymer was obtained in the same manner as in Example 11 except changing the charge amount of acrylic acid to 31 parts and using 413.3 parts of Aqueous Solution (A1-3) of an oxidized polysaccharide in place of 206.7 parts of Aqueous Solution (A1-1) of an oxidized polysaccharide.

<Example 16> Production of Hydrous Gel (G16) of Graft Polymer:

Hydrous Gel (G16) of a graft polymer was obtained in the same manner as in Example 11 except using Aqueous Solution (A1-4) of an oxidized polysaccharide in place of Aqueous Solution (A1-1) of an oxidized polysaccharide.

<Example 17> Production of Hydrous Gel (G17) of Graft Polymer:

Hydrous Gel (G17) of a graft polymer was obtained in the same manner as in Example 11 except using Aqueous Solution (A1-5) of an oxidized polysaccharide in place of Aqueous Solution (A1-1) of an oxidized polysaccharide.

<Example 18> Production of Hydrous Gel (G18) of Graft Polymer:

Hydrous Gel (G18) of a graft polymer was obtained in the same manner as in Example 11 except changing the charge amount of acrylic acid to 108.5 parts and using 155.5 parts of Aqueous Solution (A1-6) of an oxidized polysaccharide in place of 206.7 parts of Aqueous Solution (A1-1) of an oxidized polysaccharide.

<Example 19> Production of Hydrous Gel (G19) of Graft Polymer:

Hydrous Gel (G19) of a graft polymer was obtained in the same manner as in Example 11 except using Aqueous Solution (A1-6) of an oxidized polysaccharide in place of Aqueous Solution (A1-1) of an oxidized polysaccharide.

<Example 20> Production of Hydrous Gel (G20) of Graft Polymer:

Hydrous Gel (G20) of a graft polymer was obtained in the same manner as in Example 11 except changing the charge amount of acrylic acid to 31 parts and using 413.3 parts of Aqueous Solution (A1-6) of an oxidized polysaccharide in place of 206.7 parts of Aqueous Solution (A1-1) of an oxidized polysaccharide.

<Example 21> Production of Hydrous Gel (G21) of Graft Polymer:

93 Parts (1.29 molar parts) of acrylic acid (produced by Mitsubishi Chemical Corporation, purity: 100%), 0.3735 parts (0.0014 molar parts) of pentaerythritol triallyl ether (produced by Daiso Co. , Ltd.) as an internal crosslinking agent, and 340.27 parts of deionized water in which 206.7 parts of Aqueous Solution (A1-1) of an oxidized polysaccharide produced in Production Example 3 was previously dissolved were kept at 3°C with stirring/mixing. After flowing nitrogen into the mixture to make a dissolved oxygen concentration of 1 ppm or less, 0.372 parts of an aqueous 1% hydrogen peroxide solution, 0.698 parts of an aqueous 2% ascorbic acid solution, and 1.395 parts of an aqueous 2% 2,2'-azobis[2-methyl-N-(2-hydroxyethyl)-propionamide] solution were added/mixed to initiate polymerization. After the temperature of the mixture reached 90°C, polymerization was allowed to proceed at 90±2°C for about 5 hours, whereby 501.01 parts of Hydrous Gel (G21) of a graft polymer was obtained.

<Example 22> Production of Hydrous Gel (G22) of Graft Polymer:

Hydrous Gel (G22) of a graft polymer was obtained in the same manner as in Example 21 except using Aqueous Solution (A1-2) of an oxidized polysaccharide in place of Aqueous Solution (A1-1) of an oxidized polysaccharide.

<Example 23> Production of Hydrous Gel (G23) of Graft Polymer:

Hydrous Gel (G23) of a graft polymer was obtained in the same manner as in Example 21 except changing the charge amount of acrylic acid to 108.5 parts and using 155.5 parts of Aqueous Solution (A1-3) of an oxidized polysaccharide in place of 206.7 parts of Aqueous Solution (A1-1) of an oxidized polysaccharide.

<Example 24> Production of Hydrous Gel (G24) of Graft Polymer:

Hydrous Gel (G24) of a graft polymer was obtained in the same manner as in Example 21 except using Aqueous Solution (A1-3) of an oxidized polysaccharide in place of Aqueous Solution (A1-1) of an oxidized polysaccharide.

<Example 25> Production of Hydrous Gel (G25) of Graft Polymer:

Hydrous Gel (G25) of a graft polymer was obtained in the same manner as in Example 21 except changing the charge amount of acrylic acid to 31 parts and using 413.3 parts of Aqueous Solution (A1-3) of an oxidized polysaccharide in place of 206.7 parts of Aqueous Solution (A1-1) of an oxidized polysaccharide.

<Example 26> Production of Hydrous Gel (G26) of Graft Polymer:

Hydrous Gel (G26) of a graft polymer was obtained in the same manner as in Example 21 except using Aqueous Solution (A1-4) of an oxidized polysaccharide in place of Aqueous Solution (A1-1) of an oxidized polysaccharide.

<Example 27> Production of Hydrous Gel (G27) of Graft Polymer:

Hydrous Gel (G27) of a graft polymer was obtained in the same manner as in Example 21 except using Aqueous Solution (A1-5) of an oxidized polysaccharide in place of Aqueous Solution (A1-1) of an oxidized polysaccharide.

<Example 28> Production of Hydrous Gel (G28) of Graft Polymer:

Hydrous Gel (G28) of a graft polymer was obtained in the same manner as in Example 21 except changing the charge amount of acrylic acid to 108.5 parts and using 155.5 parts of Aqueous Solution (A1-6) of an oxidized polysaccharide in place of 206.7 parts of Aqueous Solution (A1-1) of an oxidized polysaccharide.

<Example 29> Production of Hydrous Gel (G29) of Graft Polymer:

Hydrous Gel (G29) of a graft polymer was obtained in the same manner as in Example 21 except using Aqueous Solution (A1-6) of an oxidized polysaccharide in place of Aqueous Solution (A1-1) of an oxidized polysaccharide.

<Example 30> Production of Hydrous Gel (G30) of Graft Polymer:

Hydrous Gel (G30) of a graft polymer was obtained in the same manner as in Example 21 except changing the charge amount of acrylic acid to 31 parts and using 413.3 parts of Aqueous Solution (A1-6) of an oxidized polysaccharide in place of 206.7 parts of Aqueous Solution (A1-1) of an oxidized polysaccharide.

<Example 31> Production of Hydrous Gel (G31) of Graft Polymer:

Hydrous Gel (G31) of a graft polymer was obtained in the same manner as in Example 21 except not adding pentaerythritol triallyl ether (produced by Daiso Co., Ltd.).

<Example 32> Production of Hydrous Gel (G32) of Graft Polymer:

Hydrous Gel (G32) of a graft polymer was obtained in the same manner as in Example 31 except using Aqueous Solution (A1-2) of an oxidized polysaccharide in place of Aqueous Solution (A1-1) of an oxidized polysaccharide.

<Example 33> Production of Hydrous Gel (G33) of Graft Polymer:

Hydrous Gel (G33) of a graft polymer was obtained in the same manner as in Example 31 except changing the charge amount of acrylic acid to 108.5 parts and using 155.5 parts of Aqueous Solution (A1-3) of an oxidized polysaccharide in place of 206.7 parts of Aqueous Solution (A1-1) of an oxidized polysaccharide.

<Example 34> Production of Hydrous Gel (G34) of Graft Polymer:

Hydrous Gel (G34) of a graft polymer was obtained in the same manner as in Example 31 except using Aqueous Solution (A1-3) of an oxidized polysaccharide in place of Aqueous Solution (A1-1) of an oxidized polysaccharide.

<Example 35> Production of Hydrous Gel (G35) of Graft Polymer:

Hydrous Gel (G35) of a graft polymer was obtained in the same manner as in Example 31 except changing the charge amount of acrylic acid to 31 parts and using 413.3 parts of Aqueous Solution (A1-3) of an oxidized polysaccharide in place of 206.7 parts of Aqueous Solution (A1-1) of an oxidized polysaccharide.

<Example 36> Production of Hydrous Gel (G36) of Graft Polymer:

Hydrous Gel (G36) of a graft polymer was obtained in the same manner as in Example 31 except using Aqueous Solution (A1-4) of an oxidized polysaccharide in place of Aqueous Solution (A1-1) of an oxidized polysaccharide.

<Example 37> Production of Hydrous Gel (G37) of Graft Polymer:

Hydrous Gel (G37) of a graft polymer was obtained in the same manner as in Example 31 except using Aqueous Solution (A1-5) of an oxidized polysaccharide in place of Aqueous Solution (A1-1) of an oxidized polysaccharide.

<Example 38> Production of Hydrous Gel (G38) of Graft Polymer:

Hydrous Gel (G38) of a graft polymer was obtained in the same manner as in Example 31 except changing the charge amount of acrylic acid to 108.5 parts and using 155.5 parts of Aqueous Solution (A1-6) of an oxidized polysaccharide in place of 206.7 parts of Aqueous Solution (A1-1) of an oxidized polysaccharide.

<Example 39> Production of Hydrous Gel (G39) of Graft Polymer:

Hydrous Gel (G39) of a graft polymer was obtained in the same manner as in Example 31 except using Aqueous Solution (A1-6) of an oxidized polysaccharide in place of Aqueous Solution (A1-1) of an oxidized polysaccharide.

<Example 40> Production of Hydrous Gel (G40) of Graft Polymer:

Hydrous Gel (G40) of a graft polymer was obtained in the same manner as in Example 31 except changing the charge amount of acrylic acid to 31 parts and using 413.3 parts of Aqueous Solution (A1-6) of an oxidized polysaccharide in place of 206.7 parts of Aqueous Solution (A1-1) of an oxidized polysaccharide.

<Examples 41 to 60>

According to the charge formulation in Table 1, an aqueous 48.5 wt% sodium hydroxide solution was added and mixed while mincing a hydrous gel of (B2) a crosslinked product of polyacrylic acid and (A1) an aqueous solution of an oxidized polysaccharide or a hydrous gel of (A2) a crosslinked product of an oxidized polysaccharide by a mincer (12VR-400K manufactured by ROYAL Co.) to obtain a minced gel. The minced gel was dried in a through-flow band-type dryer (150°C, wind velocity: 2 m/sec) to obtain a dry form. The dry form was pulverized in a juicer-mixer (OSTERIZER BLENDER, manufactured by Oster Co.) and then adjusted to a particle size of 150 to 710 $\mu$m by using sieves with a sieve-opening of 150 $\mu$m and 710 $\mu$m, whereby dry form particles were obtained. While stirring 100 parts of the dry form particles at a high speed (high-speed stirring turbulizer manufactured by Hosokawa Micron Corporation; rotation speed: 2,000 rpm), a solution of surface crosslinking agent was added and mixed by spraying, and the system was allowed to stand at 150°C for 30 minutes so as to effect surface crosslinking, whereby absorbent compositions of Examples 41 to 60 were obtained. The measurement results of water-retention amount and gel elastic modulus of the obtained absorbent compositions are shown in Table 1. Here, in Table 1, the solution of surface crosslinking agent is a 2 wt% water/methanol (weight ratio = 70/30) mixed solution of ethylene glycol diglycidyl ether.

[Table 1]

EP 2 899 224 A1

| Table 1 | | Example | | | | | | | | | | | | | | | | | | | |
|---|---|---|---|---|---|---|---|---|---|---|---|---|---|---|---|---|---|---|---|---|---|
| | | 41 | 42 | 43 | 44 | 45 | 46 | 47 | 48 | 49 | 50 | 51 | 52 | 53 | 54 | 55 | 56 | 57 | 58 | 59 | 60 |
| Hydrous gel of crosslinked polyacrylic acid (B2) (parts by weight) | (B2-1) | 60 | 60 | 70 | 60 | 20 | 60 | 60 | 70 | 60 | 60 | 60 | 70 | 60 | 20 | 60 | 60 | 70 | 60 | 20 | 20 |
| Aqueous solution of oxidized polysaccharide (A1)(parts by weight) | (A1-1) | 40 | – | – | – | – | – | – | – | – | – | – | – | – | – | – | – | – | – | – | – |
| | (A1-2) | – | 40 | – | – | – | – | – | – | – | – | – | – | – | – | – | – | – | – | – | – |
| | (A1-3) | – | – | 30 | 40 | 80 | – | – | – | – | – | – | – | – | – | – | – | – | – | – | – |
| | (A1-4) | – | – | – | – | – | 40 | – | – | – | – | – | – | – | – | – | – | – | – | – | – |
| | (A1-5) | – | – | – | – | – | – | 40 | – | – | – | – | – | – | – | – | – | – | – | – | – |
| | (A1-6) | – | – | – | – | – | – | – | 30 | 40 | – | – | – | – | – | – | – | – | – | – | 80 |
| Hydrous gel of crosslinked oxidized polysaccharide (A2) (parts by weight) | (A2-1) | – | – | – | – | – | – | – | – | – | 40 | – | – | – | – | – | – | – | – | – | – |
| | (A2-2) | – | – | – | – | – | – | – | – | – | – | 40 | – | – | – | – | – | – | – | – | – |
| | (A2-3) | – | – | – | – | – | – | – | – | – | – | – | 30 | 40 | 80 | – | – | – | – | – | – |
| | (A2-4) | – | – | – | – | – | – | – | – | – | – | – | – | – | – | 40 | – | – | – | – | – |
| | (A2-5) | – | – | – | – | – | – | – | – | – | – | – | – | – | – | – | 40 | – | – | – | – |
| | (A2-6) | – | – | – | – | – | – | – | – | – | – | – | – | – | – | – | – | 30 | 40 | 80 | – |
| Aqueous 48.5 wt% NaOH solution (parts by weight) | | 18.3 | 20.9 | 17.5 | 15.3 | 6.5 | 18.9 | 19.4 | 21.5 | 20.8 | 14.6 | 14.6 | 16.8 | 14.6 | 4.8 | 14.6 | 14.6 | 16.8 | 14.6 | 4.8 | 17.1 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 37 | 38 | 38 | 35 | 34 | 37 | 37 | 37 | 38 | 34 | 37 | 36 | 34 | 33 | 35 | 36 | 37 | 36 | 35 | 33 |
| Gel elastic modulus ($\times 10^3$ N/m$^2$) | | 2.3 | 2.4 | 2.6 | 2.6 | 2 | 2.3 | 2.3 | 2.4 | 2.2 | 1.9 | 2.5 | 2.1 | 2.7 | 2.6 | 2.2 | 2.7 | 2.3 | 2.2 | 2.2 | 2 |

<Examples 61 to 80>

According to the charge formulation in Table 2, an aqueous 48.5 wt% sodium hydroxide solution was added and mixed while mincing a hydrous gel of (B2) a crosslinked product of polyacrylic acid, an aqueous solution of (A1) oxidized polysaccharide or a hydrous gel of (A2) a crosslinked product of an oxidized polysaccharide, and a binder solution by a mincer (12VR-400K manufactured by ROYAL Co.) at a temperature of 83°C to obtain a minced gel. The subsequent procedure was performed in the same manner as in Example 41 to obtain absorbent compositions of Examples 61 to 80. The measurement results of water-retention amount and gel elastic modulus of the obtained absorbent compositions are shown in Table 2. Here, in Table 2, the solution of surface crosslinking agent is a 2 wt% water/methanol (weight ratio = 70/30) mixed solution of ethylene glycol diglycidyl ether. The binder solution is a 2 wt% water/methanol (weight ratio = 70/30) mixed solution of ethylene glycol diglycidyl ether.

[Table 2]

| Table 2 | | Example | | | | | | | | | | | | | | | | | | | |
|---|---|---|---|---|---|---|---|---|---|---|---|---|---|---|---|---|---|---|---|---|---|---|
| | | 61 | 62 | 63 | 64 | 65 | 66 | 67 | 68 | 69 | 70 | 71 | 72 | 73 | 74 | 75 | 76 | 77 | 78 | 79 | 80 |
| Hydrous gel of crosslinked polyacrylic acid (B2) (parts by weight) | (B2-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution of oxidized polysaccharide (A1) (parts by weight) | (A1-1) | 40 | – | – | – | – | – | – | – | – | – | – | – | – | – | – | – | – | – | – | – |
| | (A1-2) | – | 40 | – | – | – | – | – | – | – | – | – | – | – | – | – | – | – | – | – | – |
| | (A1-3) | – | – | 30 | 40 | 80 | – | – | – | – | – | – | – | – | – | – | – | – | – | – | – |
| | (A1-4) | – | – | – | – | – | 40 | – | – | – | – | – | – | – | – | – | – | – | – | – | – |
| | (A1-5) | – | – | – | – | – | – | 40 | – | – | – | – | – | – | – | – | – | – | – | – | – |
| | (A1-6) | – | – | – | – | – | – | – | 30 | 40 | 80 | – | – | – | – | – | – | – | – | – | – |
| Hydrous gel of crosslinked oxidized polysaccharide (A2) (parts by weight) | (A2-1) | – | – | – | – | – | – | – | – | – | – | 40 | – | – | – | – | – | – | – | – | – |
| | (A2-2) | – | – | – | – | – | – | – | – | – | – | – | 40 | – | – | – | – | – | – | – | – |
| | (A2-3) | – | – | – | – | – | – | – | – | – | – | – | – | 30 | 40 | 80 | – | – | – | – | – |
| | (A2-4) | – | – | – | – | – | – | – | – | – | – | – | – | – | – | – | 40 | – | – | – | – |
| | (A2-5) | – | – | – | – | – | – | – | – | – | – | – | – | – | – | – | – | 40 | – | – | – |
| | (A2-6) | – | – | – | – | – | – | – | – | – | – | – | – | – | – | – | – | – | 30 | 40 | 80 |
| Solution of binder | | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Aqueous 48.5 wt% NaOH solution (parts by weight) | | 18.3 | 20.9 | 17.5 | 15.3 | 6.5 | 18.9 | 19.4 | 21.5 | 20.8 | 17.1 | 14.6 | 14.6 | 16.8 | 14.6 | 4.8 | 14.6 | 14.6 | 16.8 | 14.6 | 4.8 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 36 | 37 | 35 | 35 | 32 | 36 | 37 | 38 | 36 | 34 | 36 | 35 | 34 | 32 | 34 | 35 | 36 | 35 | 34 | 31 |
| Gel elastic modulus ($\times 10^3$ N/m$^2$) | | 2.5 | 2.1 | 2.7 | 2.6 | 2.4 | 2.7 | 2.3 | 2.4 | 2.4 | 1.9 | 2.6 | 2.3 | 2.4 | 2.3 | 2 | 2.2 | 2.7 | 2.6 | 2.6 | 2.1 |

<Examples 81 to 100>

According to the charge formulation in Table 3, an aqueous 48.5 wt% sodium hydroxide solution was added and mixed while mincing an aqueous solution of (B1) polyacrylic acid and an aqueous solution of (A1) oxidized polysaccharide or a hydrous gel of (A2) a crosslinked product of an oxidized polysaccharide by a mincer (12VR-400K manufactured by ROYAL Co.) to obtain a minced gel. The subsequent procedure was performed in the same manner as in Example 41 to obtain absorbent compositions of Examples 81 to 100. The measurement results of water-retention amount and gel elastic modulus of the obtained absorbent compositions are shown in Table 3. Here, in Table 3, the solution of surface crosslinking agent is a 2 wt% water/methanol (weight ratio = 70/30) mixed solution of ethylene glycol diglycidyl ether.

[Table 3]

| Table 3 | | Example | | | | | | | | | | | | | | | | | | | |
|---|---|---|---|---|---|---|---|---|---|---|---|---|---|---|---|---|---|---|---|---|---|
| | | 81 | 82 | 83 | 84 | 85 | 86 | 87 | 88 | 89 | 90 | 91 | 92 | 93 | 94 | 95 | 96 | 97 | 98 | 99 | 100 |
| Aqueous solution of polyacrylic acid (B1) (parts by weight) | (B1-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution of oxidized polysaccharide (A1) (parts by weight) | (A1-1) | 40 | – | – | – | – | – | – | – | – | – | – | – | – | – | – | – | – | – | – | – |
| | (A1-2) | – | 40 | – | – | – | – | – | – | – | – | – | – | – | – | – | – | – | – | – | – |
| | (A1-3) | – | – | 30 | 40 | 80 | – | – | – | – | – | – | – | – | – | – | – | – | – | – | – |
| | (A1-4) | – | – | – | – | – | 40 | – | – | – | – | – | – | – | – | – | – | – | – | – | – |
| | (A1-5) | – | – | – | – | – | – | 40 | – | – | – | – | – | – | – | – | – | – | – | – | – |
| | (A1-6) | – | – | – | – | – | – | – | 30 | 40 | 80 | – | – | – | – | – | – | – | – | – | – |
| Hydrous gel of crosslinked oxidized polysaccharide (A2) (parts by weight) | (A2-1) | – | – | – | – | – | – | – | – | – | – | 40 | – | – | – | – | – | – | – | – | – |
| | (A2-2) | – | – | – | – | – | – | – | – | – | – | – | 40 | – | – | – | – | – | – | – | – |
| | (A2-3) | – | – | – | – | – | – | – | – | – | – | – | – | 30 | 40 | 80 | – | – | – | – | – |
| | (A2-4) | – | – | – | – | – | – | – | – | – | – | – | – | – | – | – | 40 | – | – | – | – |
| | (A2-5) | – | – | – | – | – | – | – | – | – | – | – | – | – | – | – | – | 40 | – | – | – |
| | (A2-6) | – | – | – | – | – | – | – | – | – | – | – | – | – | – | – | – | – | 30 | 40 | 80 |
| Aqueous 48.5 wt% NaOH solution (parts by weight) | | 18.3 | 20.9 | 17.5 | 15.3 | 6.5 | 18.9 | 19.4 | 21.5 | 20.8 | 17.1 | 14.6 | 14.6 | 16.8 | 14.6 | 4.8 | 14.6 | 14.6 | 16.8 | 14.6 | 4.8 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 39 | 41 | 40 | 37 | 35 | 37 | 39 | 41 | 40 | 36 | 38 | 37 | 36 | 34 | 36 | 37 | 38 | 37 | 35 | 33 |
| Gel elastic modulus ($\times 10^3$ N/m²) | | 2.1 | 2.1 | 2.2 | 2 | 1.8 | 1.9 | 1.9 | 2 | 1.8 | 1.7 | 1.8 | 2.1 | 2.1 | 2 | 1.9 | 2 | 2.1 | 2.1 | 2.1 | 1.7 |

EP 2 899 224 A1

24

<Examples 101 to 120>

According to the charge formulation in Table 4, an aqueous 48.5 wt% sodium hydroxide solution was added and mixed while mincing an aqueous solution of (B1) polyacrylic acid, an aqueous solution of (A1) oxidized polysaccharide or a hydrous gel of (A2) a crosslinked product of an oxidized polysaccharide, and a binder solution at a temperature of 80°C by a mincer (12VR-400K manufactured by ROYAL Co.) to obtain a minced gel. The subsequent procedure was performed in the same manner as in Example 41 to obtain absorbent compositions of Examples 101 to 120. The measurement results of water-retention amount and gel elastic modulus of the obtained absorbent compositions are shown in Table 4. Here, in Table 4, the solution of surface crosslinking agent is a 2 wt% water/methanol (weight ratio = 70/30) mixed solution of ethylene glycol diglycidyl ether. The binder solution is a 2 wt% water/methanol (weight ratio = 70/30) mixed solution of ethylene glycol diglycidyl ether.

[Table 4]

| Table 4 | | Example | | | | | | | | | | | | | | | | | | | |
|---|---|---|---|---|---|---|---|---|---|---|---|---|---|---|---|---|---|---|---|---|---|
| | | 101 | 102 | 103 | 104 | 105 | 106 | 107 | 108 | 109 | 110 | 111 | 112 | 113 | 114 | 115 | 116 | 117 | 118 | 119 | 120 |
| Aqueous solution of polyacrylic acid (B1) (parts by weight) | (B1-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution of oxidized polysaccharide (A1) (parts by weight) | (A1-1) | 40 | – | – | – | – | – | – | – | – | – | – | – | – | – | – | – | – | – | – | – |
| | (A1-2) | – | 40 | – | – | – | – | – | – | – | – | – | – | – | – | – | – | – | – | – | – |
| | (A1-3) | – | – | 30 | 40 | 80 | – | – | – | – | – | – | – | – | – | – | – | – | – | – | – |
| | (A1-4) | – | – | – | – | – | 40 | – | – | – | – | – | – | – | – | – | – | – | – | – | – |
| | (A1-5) | – | – | – | – | – | – | 40 | – | – | – | – | – | – | – | – | – | – | – | – | – |
| | (A1-6) | – | – | – | – | – | – | – | 30 | 40 | 80 | – | – | – | – | – | – | – | – | – | – |
| Hydrous gel of crosslinked oxidized polysaccharide (A2) (parts by weight) | (A2-1) | – | – | – | – | – | – | – | – | – | – | 40 | – | – | – | – | – | – | – | – | – |
| | (A2-2) | – | – | – | – | – | – | – | – | – | – | – | 40 | – | – | – | – | – | – | – | – |
| | (A2-3) | – | – | – | – | – | – | – | – | – | – | – | – | 30 | 40 | 80 | – | – | – | – | – |
| | (A2-4) | – | – | – | – | – | – | – | – | – | – | – | – | – | – | – | 40 | – | – | – | – |
| | (A2-5) | – | – | – | – | – | – | – | – | – | – | – | – | – | – | – | – | 40 | – | – | – |
| | (A2-6) | – | – | – | – | – | – | – | – | – | – | – | – | – | – | – | – | – | 30 | 40 | 80 |
| Solution of binder | | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Aqueous 48.5 wt% NaOH solution (parts by weight) | | 18.3 | 20.9 | 17.5 | 15.3 | 6.5 | 18.9 | 19.4 | 21.5 | 20.8 | 17.1 | 14.6 | 14.6 | 16.8 | 14.6 | 4.8 | 14.6 | 14.6 | 16.8 | 14.6 | 4.8 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 38 | 39 | 39 | 36 | 33 | 37 | 38 | 40 | 38 | 35 | 36 | 35 | 34 | 33 | 32 | 36 | 36 | 36 | 36 | 33 |
| Gel elastic modulus ($\times 10^3$ N/m$^2$) | | 2.2 | 2.5 | 2.2 | 2.1 | 1.9 | 2.2 | 2.3 | 2.1 | 2 | 1.7 | 1.9 | 1.9 | 2.2 | 2 | 2 | 2.3 | 2.1 | 2.2 | 2.2 | 2 |

EP 2 899 224 A1

<Examples 121 to 160>

An aqueous 48.5 wt% sodium hydroxide solution was added and mixed while mincing a hydrous gel of (G) graft polymer by a mincer (12VR-400K manufactured by ROYAL Co.) to obtain a minced gel. The subsequent procedure was performed in the same manner as in Example 41 to obtain absorbent compositions of Examples 121 to 160. The measurement results of water-retention amount and gel elastic modulus of the obtained absorbent compositions are shown in Tables 5-1 and 5-2. Here, in Tables 5-1 and 5-2, the solution of surface crosslinking agent is a 2 wt% water/methanol (weight ratio = 70/30) mixed solution of ethylene glycol diglycidyl ether.

[Table 5-1]

| Table 5-1 | | Example | | | | | | | | | | | | | | | | | | |
|---|---|---|---|---|---|---|---|---|---|---|---|---|---|---|---|---|---|---|---|---|
| | | 121 | 122 | 123 | 124 | 125 | 126 | 127 | 128 | 129 | 130 | 131 | 132 | 133 | 134 | 135 | 136 | 137 | 138 | 139 | 140 |
| Hydrous gel of graft polymer (G) (parts by weight) | (G1) | 100 | – | – | – | – | – | – | – | – | – | – | – | – | – | – | – | – | – | – | – |
| | (G2) | – | 100 | – | – | – | – | – | – | – | – | – | – | – | – | – | – | – | – | – | – |
| | (G3) | – | – | 100 | – | – | – | – | – | – | – | – | – | – | – | – | – | – | – | – | – |
| | (G4) | – | – | – | 100 | – | – | – | – | – | – | – | – | – | – | – | – | – | – | – | – |
| | (G5) | – | – | – | – | 100 | – | – | – | – | – | – | – | – | – | – | – | – | – | – | – |
| | (G6) | – | – | – | – | – | 100 | – | – | – | – | – | – | – | – | – | – | – | – | – | – |
| | (G7) | – | – | – | – | – | – | 100 | – | – | – | – | – | – | – | – | – | – | – | – | – |
| | (G8) | – | – | – | – | – | – | – | 100 | – | – | – | – | – | – | – | – | – | – | – | – |
| | (G9) | – | – | – | – | – | – | – | – | 100 | – | – | – | – | – | – | – | – | – | – | – |
| | (G10) | – | – | – | – | – | – | – | – | – | 100 | – | – | – | – | – | – | – | – | – | – |
| | (G11) | – | – | – | – | – | – | – | – | – | – | 100 | – | – | – | – | – | – | – | – | – |
| | (G12) | – | – | – | – | – | – | – | – | – | – | – | 100 | – | – | – | – | – | – | – | – |
| | (G13) | – | – | – | – | – | – | – | – | – | – | – | – | 100 | – | – | – | – | – | – | – |
| | (G14) | – | – | – | – | – | – | – | – | – | – | – | – | – | 100 | – | – | – | – | – | – |
| | (G15) | – | – | – | – | – | – | – | – | – | – | – | – | – | – | 100 | – | – | – | – | – |
| | (G16) | – | – | – | – | – | – | – | – | – | – | – | – | – | – | – | 100 | – | – | – | – |
| | (G17) | – | – | – | – | – | – | – | – | – | – | – | – | – | – | – | – | 100 | – | – | – |
| | (G18) | – | – | – | – | – | – | – | – | – | – | – | – | – | – | – | – | – | 100 | – | – |
| | (G19) | – | – | – | – | – | – | – | – | – | – | – | – | – | – | – | – | – | – | 100 | – |
| | (G20) | – | – | – | – | – | – | – | – | – | – | – | – | – | – | – | – | – | – | – | 100 |
| Aqueous 48.5 wt% NaOH solution (parts by weight) | | 18.3 | 20.9 | 17.5 | 15.3 | 6.5 | 18.9 | 19.4 | 21.5 | 20.8 | 17.1 | 18.3 | 20.9 | 17.5 | 15.3 | 6.5 | 18.9 | 19.4 | 21.5 | 20.8 | 17.1 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 36 | 38 | 36 | 36 | 34 | 37 | 36 | 37 | 35 | 35 | 37 | 38 | 37 | 37 | 35 | 39 | 38 | 37 | 38 | 37 |
| Gel elastic modulus ($\times 10^3$ N/m$^2$) | | 2.3 | 2.1 | 2.2 | 2.2 | 2.1 | 2.2 | 2.3 | 2.4 | 2.4 | 2 | 2 | 2 | 2 | 1.9 | 1.7 | 1.9 | 2.1 | 2 | 2 | 1.8 |

[Table 5-2]

| Table 5-2 | | Example | | | | | | | | | | | | | | | | | | | |
|---|---|---|---|---|---|---|---|---|---|---|---|---|---|---|---|---|---|---|---|---|---|
| | | 141 | 142 | 143 | 144 | 145 | 146 | 147 | 148 | 149 | 150 | 151 | 152 | 153 | 154 | 155 | 156 | 157 | 158 | 159 | 160 |
| Hydrous gel of graft polymer (G) (parts by weight) | (G21) | 100 | – | – | – | – | – | – | – | – | – | – | – | – | – | – | – | – | – | – | – |
| | (G22) | – | 100 | – | – | – | – | – | – | – | – | – | – | – | – | – | – | – | – | – | – |
| | (G23) | – | – | 100 | – | – | – | – | – | – | – | – | – | – | – | – | – | – | – | – | – |
| | (G24) | – | – | – | 100 | – | – | – | – | – | – | – | – | – | – | – | – | – | – | – | – |
| | (G25) | – | – | – | – | 100 | – | – | – | – | – | – | – | – | – | – | – | – | – | – | – |
| | (G26) | – | – | – | – | – | 100 | – | – | – | – | – | – | – | – | – | – | – | – | – | – |
| | (G27) | – | – | – | – | – | – | 100 | – | – | – | – | – | – | – | – | – | – | – | – | – |
| | (G28) | – | – | – | – | – | – | – | 100 | – | – | – | – | – | – | – | – | – | – | – | – |
| | (G29) | – | – | – | – | – | – | – | – | 100 | – | – | – | – | – | – | – | – | – | – | – |
| | (G30) | – | – | – | – | – | – | – | – | – | 100 | – | – | – | – | – | – | – | – | – | – |
| | (G31) | – | – | – | – | – | – | – | – | – | – | 100 | – | – | – | – | – | – | – | – | – |
| | (G32) | – | – | – | – | – | – | – | – | – | – | – | 100 | – | – | – | – | – | – | – | – |
| | (G33) | – | – | – | – | – | – | – | – | – | – | – | – | 100 | – | – | – | – | – | – | – |
| | (G34) | – | – | – | – | – | – | – | – | – | – | – | – | – | 100 | – | – | – | – | – | – |
| | (G35) | – | – | – | – | – | – | – | – | – | – | – | – | – | – | 100 | – | – | – | – | – |
| | (G36) | – | – | – | – | – | – | – | – | – | – | – | – | – | – | – | 100 | – | – | – | – |
| | (G37) | – | – | – | – | – | – | – | – | – | – | – | – | – | – | – | – | 100 | – | – | – |
| | (G38) | – | – | – | – | – | – | – | – | – | – | – | – | – | – | – | – | – | 100 | – | – |
| | (G39) | – | – | – | – | – | – | – | – | – | – | – | – | – | – | – | – | – | – | 100 | – |
| | (G40) | – | – | – | – | – | – | – | – | – | – | – | – | – | – | – | – | – | – | – | 100 |
| Aqueous 48.5 wt% NaOH solution (parts by weight) | | 18.3 | 20.9 | 17.5 | 15.3 | 6.5 | 18.9 | 19.4 | 21.5 | 20.8 | 17.1 | 18.3 | 20.9 | 17.5 | 15.3 | 6.5 | 18.9 | 19.4 | 21.5 | 20.8 | 17.1 |
| Solution of surface crosslinking agent (parts by weight) | | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | | 37 | 40 | 38 | 38 | 37 | 38 | 37 | 38 | 36 | 37 | 38 | 40 | 39 | 39 | 38 | 41 | 40 | 39 | 40 | 39 |
| Gel elastic modulus ($\times 10^3$ N/m$^2$) | | 2.3 | 2 | 2.1 | 2.1 | 2 | 1.7 | 1.7 | 2.1 | 2.2 | 2.2 | 2 | 1.9 | 1.9 | 2.1 | 2 | 1.6 | 2 | 1.9 | 2 | 2 |

<Comparative Example 1>

An absorbent composition for comparison was obtained in the same manner as in Example 41 except using a 30 wt% aqueous solution of commercially available starch (SK-100 produced by Japan Corn Starch Co. , Ltd.) in place of Aqueous Solution (A1-1) of an oxidized polysaccharide and changing the charge amount of an aqueous 48.5 wt% sodium hydroxide solution to 16.8 parts.

<Comparative Example 2>

An absorbent composition for comparison was obtained in the same manner as in Example 41 except using a 30 wt% aqueous solution of commercially available corn starch (produced by Wako Pure Chemical Industries, Ltd.) in place of Aqueous Solution (A1-1) of an oxidized polysaccharide and changing the charge amount of an aqueous 48.5 wt% sodium hydroxide solution to 16.8 parts.

<Comparative Example 3>

An absorbent composition for comparison was obtained in the same manner as in Example 41 except using a 30 wt% aqueous solution of commercially available Kiprogum (M-800A, produced by Nippon Starch Chemical Co., Ltd.) in place of Aqueous Solution (A1-1) of an oxidized polysaccharide and changing the charge amount of an aqueous 48.5 wt% sodium hydroxide solution to 16.8 parts.

<Comparative Example 4>

An absorbent composition for comparison was obtained in the same manner as in Example 61 except using a 30 wt% aqueous solution of commercially available carboxymethyl cellulose (produced by Aldrich Chemical Co. Inc., substitution degree: 0.7) in place of Aqueous Solution (A1-1) of an oxidized polysaccharide and changing the charge amount of an aqueous 48.5 wt% sodium hydroxide solution to 20.5 parts.

<Comparative Example 5>

An absorbent composition for comparison was obtained in the same manner as in Example 61 except using 100 parts of a 30 wt% aqueous solution of commercially available carboxymethyl cellulose (produced by Aldrich Chemical Co. Inc., substitution degree: 0.7) in place of 60 parts of Hydrous Gel (B2-1) of a crosslinked polyacrylic acid and 40 parts of Aqueous Solution (A1-1) of an oxidized polysaccharide and changing the charge amount of an aqueous 48.5 wt% sodium hydroxide solution to 12.2 parts.

<Comparative Example 6>

An absorbent composition for comparison was obtained in the same manner as in Example 41 except using 100 parts of a 30 wt% aqueous solution of commercially available Kiprogum (M-800A, produced by Nippon Starch Chemical Co. , Ltd.) in place of 60 parts of Hydrous Gel (B2-1) of a crosslinked polyacrylic acid and 40 parts of Aqueous Solution (A1-1) of an oxidized polysaccharide and changing the charge amount of an aqueous 48.5 wt% sodium hydroxide solution to 2.2 parts. The measurement results of water-retention amount and gel elastic modulus of the absorbent compositions for comparison obtained in

Comparative Examples 1 to 6 are shown in Table 6.

[Table 6]

| Table 6 | | Waster-retention amount (g/g) | Gel elastic modulus ($\times 10^3$ N/m$^2$) |
|---|---|---|---|
| Comparative Example | 1 | 22 | 1.6 |
| | 2 | 19 | 1.6 |
| | 3 | 23 | 1.7 |
| | 4 | 26 | 1.6 |
| | 5 | 14 | 2.8 |
| | 6 | 8 | 1 |

It is seen from Tables 1 to 6 that as compared with the absorbent compositions of Comparative Examples 1 to 6, the absorbent compositions of Examples 1 to 160 are superior in the water-retention amount. As compared with the absorbent compositions of Comparative Examples 5 and 6, which are composed of only a polysaccharide having a carboxyl group, the absorbent compositions of Examples 1 to 160 are higher in gel elastic modulus.

<Example 161>

18.3 Parts of an aqueous 48.5 wt% sodium hydroxide solution was added and mixed while mincing 60 parts of Hydrous Gel (B2-1) of a crosslinked polyacrylic acid obtained in Production Example 1 and 40 parts of Aqueous Solution (A1-1) of an oxidized polysaccharide obtained in Production Example 3 by a mincer (12VR-400K manufactured by ROYAL Co.), and subsequently, 0.19 parts of octadecanoic acid as a hydrophobic substance was added and mixed to obtain a minced gel. The minced gel was dried in a through-flow band-type dryer (150°C, wind velocity: 2 m/sec) to obtain a dry form. The dry form was pulverized in a juicer-mixer (OSTERIZER BLENDER, manufactured by Oster Co.) and then adjusted to a particle size of 150 to 710 $\mu$m by using sieves with a sieve-opening of 150 $\mu$m and 710 $\mu$m, whereby dry form particles were obtained. While stirring 100 parts of the dry form particles at a high speed (high-speed stirring turbulizer manufactured by Hosokawa Micron Corporation; rotation speed: 2,000 rpm), 5 parts of a 2 wt% water/methanol mixed solution (weight ratio of water/methanol = 70/30) of ethylene glycol diglycidyl ether was added and mixed by spraying, and the system was allowed to stand at 150°C for 30 minutes so as to effect surface crosslinking, whereby an absorbent composition particle was obtained. The hydrophobic substance (octadecanoic acid) was present in a proportion of 0.520% in the inside of the absorbent composition particle and present in a proportion of 0.015% on the surface.

<Example 162>

An absorbent composition particle was obtained in the same manner as in Example 161 except adding 0. 38 parts of sucrose monononanoate in place of 0.19 parts of octadecanoic acid. The hydrophobic substance (sucrose monononanoate) was present in a proportion of 1.03% in the inside of the absorbent composition particle and present in a proportion of 0.061% on the surface.

<Example 163>

An absorbent composition particle was obtained in the same manner as in Example 161 except adding 0.38 parts of sorbitol monononanoate in place of 0.19 parts of octadecanoic acid. The hydrophobic substance (sorbitol mononanoate) was present in a proportion of 1.05% in the inside of the absorbent composition particle and present in a proportion of 0.035% on the surface.

<Comparative Example 7>

An absorbent composition particle for comparison was obtained in the same manner as in Example 161 except not using Aqueous Solution (A1-1) of an oxidized polysaccharide and octadecanoic acid and changing the parts used of Hydrous Gel (B2-1) of a crosslinked polyacrylic acid to 100 parts and the parts used of an aqueous 48.5 wt% sodium hydroxide solution to 24.0 parts.

The absorbent composition particles obtained in Examples 161 to 163 and Comparative Example 7 were measured for the water-retention amount, the absorption velocity by a swollen volume measuring method, and the gel elastic modulus, and the results are shown in Table 7.

[Table 7]

| Table 7 | | Content of hydrophobic substance (wt%) | | Water-retention amount (g/g) | Absorption velocity determined by swollen volume measuring method | | | Gel elastic modulus ($\times 10^3$ N/m$^2$) |
|---|---|---|---|---|---|---|---|---|
| | | Inside of particle | Surface of particle | | t1 (sec) | t2 (sec) | t2/t1 | |
| Example | 161 | 0.52 | 0.015 | 37 | 30 | 302 | 10.1 | 2.6 |
| | 162 | 1.03 | 0.061 | 36 | 33 | 336 | 10.2 | 2.4 |
| | 163 | 1.05 | 0.035 | 37 | 34 | 310 | 9.1 | 2.6 |
| Comparative Example | 7 | 0 | 0 | 38 | 19 | 577 | 30.4 | 2.4 |

It is seen from Table 7 that as compared with the absorbent composition particle of Comparative Example 7, the absorbent composition particles of Examples 161 to 163 have a fast absorption velocity in the middle and late stages and are an absorbent composition particle having an absorption velocity pattern more suitable for an absorbent article.

<Examples 164 to 166 and Comparative Example 8>

In order to examine the absorption characteristics when an absorbent composition particle having an appropriate absorption velocity pattern is applied to an absorbent article, two types of absorbent articles (disposable diaper) were prepared as follows by using each of the absorbent composition particles obtained in Examples 161 to 163 and Comparative Example 7, and the surface dryness was evaluated by the SDME method. The results are shown in Table 8.

<Preparation 1 of Absorbent Article (Disposable Diaper)>

100 Parts of fluff pulp and 100 parts of the evaluation sample (absorbent composition particle) were mixed in an air-flow type mixing apparatus (pad former manufactured by O-TEC K.K.) to obtain a mixture, and this mixture was uniformly laminated on an acryl plate (thickness: 4 mm) to have a paper weight in gsm of about 500 g/m$^2$ and then pressed under a pressure of 5 kg/cm$^2$ for 30 seconds to obtain Absorbent Material (1). Absorbent Material (1) was cut into a rectangle of 10 cm $\times$ 40 cm and after a water-absorbing paper (paper weight in gsm: 15.5 g/m$^2$, produced by Advantec Co., filter paper No. 2) having the same size as that of the absorbent material was disposed on the top and the bottom of each absorbent material, a polyethylene sheet (polyethylene film UB-1 produced by Tamapoly Co., Ltd.) and a nonwoven fabric (grams per square meter (gsm): 20 g/m$^2$, Eltas Guard produced by Asahi Kasei Corporation) were disposed on the back side and the surface, respectively, to prepare Disposable Diaper (1). The weight ratio between the absorbent composition particle and the fiber (weight of absorbent composition particle/weight of fiber) was 50/50.

<Preparation 2 of Absorbent Article (Disposable Diaper)>

Disposable Diaper (2) was prepared in the same manner as in Preparation 1 of Absorbent Article (Disposable Diaper) except changing "100 parts of fluff pulp and 100 parts of the evaluation sample (absorbent composition particle)" to "80 parts of fluff pulp and 120 parts of the evaluation sample (absorbent composition particle)". The weight ratio between the absorbent composition particle and the fiber (weight of absorbent composition particle/weight of fiber) was 60/40.

<Measurement of Surface Dryness by SDME Method>

A detector of an SDME (Surface Dryness Measurement Equipment) tester (manufactured by WK system Co.) was placed on a fully wetted disposable diaper [prepared by dipping a disposable diaper in an artificial urine (0.03 wt% of potassium chloride, 0.08 wt% of magnesium sulfate, 0.8 wt% of sodium chloride, and 99.09 wt% of deionized water) and allowing the diaper to stand for 60 minutes] to set a 0% dryness and then, the detector of the SDME tester was placed on a dry disposable diaper (prepared by drying a disposable diaper under heating at 80°C for 2 hours) to set a 100% dryness, thereby performing calibration of the SDME tester. Subsequently, a metal ring (inner diameter: 70 mm, length: 50 mm) was set on the center of the disposable diaper to be measured, and 80 ml of the artificial urine was poured therein. When absorption of the artificial urine was completed (until the gloss by the artificial urine was not confirmed), the metal ring was immediately removed and by placing three SDME detectors at the center, right side and left side of the disposable diaper (3 positions at equal intervals of 10 cm from the end of the 40 cm-wide disposable diaper), measurement of the surface dryness was started. The values 5 minutes after the start of measurement were taken as the surface dryness (center), the surface dryness (left), and the surface dryness (right).

Incidentally, the measurement was performed by setting the measurement atmosphere and the standing atmosphere at 25±5°C and 65±10% RH and the artificial urine used had been adjusted to 25±5°C.

[Table 8]

| Table 8 | | Disposable diaper (1) | | | Disposable diaper (2) | | |
| --- | --- | --- | --- | --- | --- | --- | --- |
| | | Surface dryness (%) | | | Surface dryness (%) | | |
| | | (Left) | (Center) | (Right) | (Left) | (Center) | (Right) |
| Example | 164 | 87 | 88 | 90 | 88 | 90 | 91 |
| | 165 | 89 | 87 | 91 | 82 | 89 | 80 |
| | 166 | 90 | 90 | 86 | 81 | 80 | 82 |
| Comparative Example | 8 | 79 | 64 | 65 | 80 | 65 | 60 |

As seen from Table 8, the disposable diapers using the absorbent composition particles of Examples 164 to 166 were excellent with less variation among the surface dryness (center), (left) and (right), as compared with the disposable diaper using the absorbent composition particle of Comparative Example 8. That is, thanks to a more appropriate absorption velocity pattern, the absorbent composition particle of the present invention exhibited excellent absorption characteristics when applied to an absorbent article. Accordingly, it may be easily expected that even when an absorbent article to which the absorbent composition particle of the present invention is applied is used, there is no fear of skin irritation and the like.

INDUSTRIAL APPLICABILITY

The absorbent resin, absorbent resin particle, absorbent composition and absorbent composition particle of the present invention have a high plant-derived raw material proportion and are excellent in absorption capacity for an aqueous liquid and by virtue of having good gel elastic modulus, also excellent in absorption capacity under pressure, and therefore, these are useful for various industrial applications such as hygienic material (e.g., disposable diaper for child and adult, sanitary napkin, hygienic cotton, bandage, incontinence pad, paper towel), a material for food industry (e.g., freshness-keeping agent, drip absorbing agent), a garden material (e.g., water retention agent, soil conditioner), and a building material (e.g., anti-dewing agent).

DESCRIPTION OF REFERENCE SIGNS

1: Bottomed cylinder
2: Disk
3: Absorbent composition particle
4 : Indication part for thickness measurement of Digimatic Indicator
5: Rod of Digimatic Indicator
6: Platform of Digimatic Indicator

Claims

1. An aqueous liquid absorbing resin comprising (A1) an oxidized polysaccharide having a carboxyl group optionally neutralized by a neutralizing agent and/or (A2) a crosslinked product thereof and (B1) a poly (meth) acrylic acid with at least one of the carboxyl groups thereof optionally being neutralized by a neutralizing agent and/or (B2) a crosslinked product thereof, the (A1) and/or the (A2) being bonded with the (B1) and/or the (B2), wherein the weight average molecular weight of the (A1) is 2,000 to 10,000,000, the acid value of the (A1) is 65 to 524 mgKOH/g when the (A1) has no carboxyl groups neutralized by a neutralizing agent, and the acid value of the (A1) prior to its neutralization is 65 to 524 mgKOH/g when the (A1) has a carboxyl group neutralized by a neutralizing agent.

2. The aqueous liquid absorbing resin according to claim 1, wherein the (A1) is an oxidized starch or an oxidized cellulose each having a carboxyl group optionally neutralized by a neutralizing agent.

3. The aqueous liquid absorbing resin according to claim 1 or 2, wherein the (A1) and/or the (A2) are bonded with the (B1) and/or the (B2) using at least one binder selected from the group consisting of (k1) a polyhydric alcohol having 2 to 6 carbon atoms, (k2) a polyvalent glycidyl ether having 8 to 21 carbon atoms, (k3) a polyvalent amine having 2 to 6 carbon atoms, and (k4) an alkanolamine having 2 to 8 carbon atoms.

4. The aqueous liquid absorbing resin according to claim 1 or 2, wherein the (A1) and/or the (A2) are bonded with the (B1) and/or the (B2) by graft polymerizing the (B1) and/or the (B2) to the (A1) and/or the (A2).

5. The aqueous liquid absorbing resin according to any one of claims 1 to 4, wherein the neutralization ratio to the total amount of the carboxyl groups possessed by the (B1) and the (B2) is less than 85%.

6. The aqueous liquid absorbing resin according to any one of claims 1 to 5, wherein the neutralization ratio of the total amount of the carboxyl groups possessed by the (A1), the (A2), the (B1) and the (B2) is 65 to 75%.

7. The aqueous liquid absorbing resin according to any one of claims 1 to 6, wherein the neutralizing agent is a hydroxide of an alkali metal.

8. The aqueous liquid absorbing resin according to any one of claims 1 to 7, wherein the total amount of the (A1) and the (A2) bound relative to the weight of the absorbent resin is 30 to 90 wt%.

9. An absorbent resin particle comprising the aqueous liquid absorbing resin according to any one of claims 1 to 8 and a hydrophobic substance (E), wherein the (E) is a compound having at least one monovalent aliphatic hydrocarbon group having 8 to 26 carbon atoms and having at least one functional group capable of forming a hydrogen bond with a carboxyl group, and the (E) is present in the inside of the absorbent resin particle in an amount of 0.01 to 10.0 wt% based on the weight of the aqueous liquid absorbing resin and on the surface of the absorbent resin particle in an amount of 0.001 to 1.0 wt%.

10. The absorbent resin particle according to claim 9, wherein the ratio (t2/t1) of the time (t1) taken until 1 g of the absorbent resin particle absorbs a physiological saline solution and the swollen volume of the particle reaches 5 ml to the time (t2) taken until the swollen volume reaches 40 ml is from 5 to 20 at 25°C.

11. The absorbent resin particle according to claim 9 or 10, wherein the functional group capable of forming a hydrogen bond with a carboxyl group is at least one functional group selected from the group consisting of a carboxyl group, a phosphoric acid group, a sulfonic acid group, a primary amino group, a secondary amino group, a tertiary amino group, a hydroxyl group, an oxycarbonyl group, a phosphate group, a sulfonate group, an amide group, a urethane group, and a urea group.

12. An absorbent material produced using the aqueous liquid absorbing resin according to any one of claims 1 to 8 and/or the absorbent resin particle according to any one of claims 9 to 11, and at least one species selected from the group consisting of a fiber, a nonwoven fabric, and a woven fabric.

13. An absorbent article produced using the absorbent material according to claim 12.

14. An aqueous liquid absorbing composition comprising (A1) an oxidized polysaccharide having a carboxyl group optionally neutralized by a neutralizing agent and/or (A2) a crosslinked product thereof and (B1) a poly (meth) acrylic acid with at least one of the carboxyl groups thereof optionally being neutralized by a neutralizing agent and/or (B2) a crosslinked product thereof, wherein the weight average molecular weight of the (A1) is 2,000 to 10,000,000, the acid value of the (A1) is 65 to 524 mgKOH/g when the (A1) has no carboxyl groups neutralized by a neutralizing agent, and the acid value of the (A1) prior to its neutralization is 65 to 524 mgKOH/g when the (A1) has a carboxyl group neutralized by a neutralizing agent.

15. The aqueous liquid absorbing composition according to claim 14, further comprising the aqueous liquid absorbing resin according to any one of claims 1 to 8.

16. The aqueous liquid absorbing composition according to claim 14 or 15, wherein the (A1) is an oxidized starch or an oxidized cellulose each having a carboxyl group optionally neutralized by a neutralizing agent.

17. The aqueous liquid absorbing composition according to any one of claims 14 to 16, wherein the neutralization ratio to the total amount of the carboxyl groups possessed by the (B1) and the (B2) is less than 85%.

18. The aqueous liquid absorbing composition according to any one of claims 14 to 17, wherein the neutralization ratio of the total amount of the carboxyl groups possessed by the (A1), the (A2), the (B1) and the (B2) is 65 to 75%.

19. The aqueous liquid absorbing composition according to any one of claims 14 to 18, wherein the neutralizing agent is a hydroxide of an alkali metal.

20. The aqueous liquid absorbing composition according to any one of claims 14 to 19, wherein when containing no aqueous liquid absorbing resin, the total amount of the (A1) and the (A2) contained is from 30 to 90 wt% based on the weight of the aqueous liquid absorbing composition, and when containing an aqueous liquid absorbing resin, the total amount of the (A1) and the (A2) contained and the (A1) and the (A2) bound in the aqueous liquid absorbing resin is from 30 to 90 wt% based on the weight of the aqueous liquid absorbing composition.

21. An absorbent composition particle comprising the aqueous liquid absorbing composition according to any one of claims 14 to 20 and a hydrophobic substance (E), wherein the (E) is a compound having at least one monovalent aliphatic hydrocarbon group having 8 to 26 carbon atoms and having at least one functional group capable of forming a hydrogen bond with a carboxyl group, and the (E) is present in the inside of the absorbent composition particle in an amount of 0.01 to 10.0 wt% based on the weight of the absorbent composition particle and on the surface of the absorbent composition particle in an amount of 0.001 to 1.0 wt%.

22. The absorbent composition particle according to claim 21, wherein the ratio (t2/t1) of the time (t1) taken until 1 g of the absorbent composition particle absorbs a physiological saline solution and the swollen volume of the particle reaches 5 ml to the time (t2) taken until the swollen volume reaches 40 ml is from 5 to 20 at 25°C.

23. The absorbent composition particle according to claim 21 or 22, wherein the functional group capable of forming a hydrogen bond with a carboxyl group is at least one functional group selected from the group consisting of a carboxyl group, a phosphoric acid group, a sulfonic acid group, a primary amino group, a secondary amino group, a tertiary amino group, a hydroxyl group, an oxycarbonyl group, a phosphate group, a sulfonate group, an amide group, a urethane group, and a urea group.

24. An absorbent material produced using the aqueous liquid absorbing composition according to any one of claims 14 to 20 and/or the absorbent composition particle according to any one of claims 21 to 23, and at least one species selected from the group consisting of a fiber, a nonwoven fabric, and a woven fabric.

25. An absorbent article produced using the absorbent material according to claim 24.

FIG.1

## INTERNATIONAL SEARCH REPORT

| | International application No. |
|---|---|
| | PCT/JP2013/075106 |

A. CLASSIFICATION OF SUBJECT MATTER
*C08G81/02*(2006.01)i, *C08F251/00*(2006.01)i, *C08K5/00*(2006.01)i, *C08L51/02* (2006.01)i

According to International Patent Classification (IPC) or to both national classification and IPC

B. FIELDS SEARCHED

Minimum documentation searched (classification system followed by classification symbols)
C08G81/00-81/02, C08F251/00-251/02, C08K5/00-5/59, C08L51/00-51/10

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-303319 A (San-Dia Polymers, Inc.), 18 December 2008 (18.12.2008), entire text (Family: none) | 1-25 |
| A | JP 52-82715 A (Sanyo Chemical Industries, Ltd.), 11 July 1977 (11.07.1977), entire text (Family: none) | 1-25 |
| A | JP 53-149545 A (Sanyo Chemical Industries, Ltd.), 27 December 1978 (27.12.1978), entire text (Family: none) | 1-25 |

| [X] Further documents are listed in the continuation of Box C. | [ ] See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 October, 2013 (07.10.13) | 15 October, 2013 (15.10.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/075106 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 51-125683 A  (Sanyo Chemical Industries, Ltd.),<br>02 November 1976 (02.11.1976),<br>entire text<br>(Family: none) | 1-25 |
| A | JP 60-4583 A  (Toppan Printing Co., Ltd.),<br>11 January 1985 (11.01.1985),<br>entire text<br>(Family: none) | 1-25 |
| A | JP 61-7316 A  (Sanyo Electric Co., Ltd., Tokyo Sanyo Electric Co., Ltd.),<br>14 January 1986 (14.01.1986),<br>entire text<br>(Family: none) | 1-25 |
| A | JP 3-174414 A  (Sanyo Chemical Industries, Ltd., Hoechst Celanese Corp.),<br>29 July 1991 (29.07.1991),<br>entire text<br>& JP 10-265522 A         & US 5453323 A1<br>& US 5145906 A1 | 1-25 |
| A | JP 3-177431 A  (Sanyo Chemical Industries, Ltd.),<br>01 August 1991 (01.08.1991),<br>entire text<br>& US 5112903 A          & EP 407147 A2<br>& DE 69030844 C          & DE 69030844 T | 1-25 |
| A | JP 1-285119 A  (Sanyo Chemical Industries, Ltd.),<br>16 November 1989 (16.11.1989),<br>entire text<br>(Family: none) | 1-25 |
| A | JP 2007-270152 A  (Stockhausen GmbH),<br>18 October 2007 (18.10.2007),<br>entire text<br>(Family: none) | 1-25 |
| P,X | WO 2012/128264 A1  (Sanyo Chemical Industries, Ltd.),<br>27 September 2012 (27.09.2012),<br>claims 1 to 25; paragraphs [0001], [0006], [0008], [0015], [0209]; examples; tables 1 to 9<br>& JP 5131945 B            & JP 2013-17831 A<br>& TW 201302896 A | 1-25 |

Form PCT/ISA/210 (continuation of second sheet) (July 2009)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0489424 A
- JP 62247837 A
- JP 10195101 A
- JP 3205168 B
- JP 2003225565 A
- JP 2006131767 A
- JP 59189103 A
- JP 61211305 A
- JP 61252212 A
- JP 51136588 A
- JP 61257235 A
- JP 2011252088 A

**Non-patent literature cited in the description**

- **F. MASUDA.** *Chemical Economy & Engineering Review,* November 1983, 19
- Perrys Chemical Engineer's Handbook. McGraw-Hill Book Company, 1984, 21